# FASCICULE DE BREVET EUROPEEN

(11) **EP 3 897 682 B1**
(45) Date de publication et mention de la délivrance du brevet: **26.10.2022**
(21) Numéro de dépôt: 19832611.8
(22) Date de dépôt: 17.12.2019
(51) Int. Cl.: A61K 36/064, A61K 9/00, A61K 47/02, A61P 31/00, A61P 1/02, A61K 9/08

(54) **SOUCHE DE LEVURE SACCHAROMYCES CEREVISIAE POUR LE TRAITEMENT ET/OU LA PRÉVENTION DE CANDIDOSES OROPHARYNGÉES**
SACCHAROMYCES CEREVISIAE STAMM ZUR BEHANDLUNG VON OROPHARYNGEALEN CANDIDOSE
SACCHAROMYCES CEREVISIAE STRAIN FOR TREATING OROPHARYNGEAL CANDIDOSIS

(30) Priorité: 17.12.2018 FR 1873109
(43) Date de publication de la demande: 27.10.2021
(73) Titulaire: Lesaffre et Compagnie, 75001 Paris (FR)
(72) Inventeur: BALLET, Nathalie, 59700 MARC EN BAROEUL (FR); DECHERF, Amélie, 59910 BONDUES (FR)
(74) Mandataire: Plasseraud IP
(86) Numéro de dépôt international: PCT/EP2019/085473
(87) Numéro de publication internationale: WO 2020/127136

(56) Documents cités:
- WO-A1-2014/009656
- WO-A2-2009/103884
- REZA MOHAMMAD SALEHI ET AL: "Effect of Saccharomyces boulardii Extract on SAP2 Gene Expression and Antifungal Susceptibility of Candida albicans", JUNDISHAPUR JOURNAL OF MICROBIOLOGY, vol. 11, no. 3, 17 février 2018 (2018-02-17), page 59891, XP055629367, ISSN: 2008-3645, DOI: 10.5812/jjm.59891
- TIMOTHY J BREAK ET AL: "VT-1161 protects mice against oropharyngeal candidiasis caused by fluconazole-susceptible and -resistant Candida albicans", JOURNAL OF ANTIMICROBIAL CHEMOTHERAPY., vol. 73, no. 1, 1 janvier 2018 (2018-01-01), pages 151-155, XP055629372, GB ISSN: 0305-7453, DOI: 10.1093/jac/dkx352

## Description

### Domaine de l'Invention

La présente invention concerne le domaine des infections oropharyngées par *Candida.* L'invention porte plus particulièrement sur une souche spécifique de *Saccharomyces cerevisiae* utile dans le traitement et/ou la prévention de candidoses buccales et oropharyngées.

### Contexte de l'Invention

La candidose oropharyngée est une infection mucosale très fréquente causée, dans la plupart des cas, par le champignon *Candida albicans,* même si d'autres espèces, telles que *C. glabrata*, *C. pseudotropicalis, C. krusei, C. parakrusei, et C. guillermondii,* peuvent aussi être à l'origine de cette mycose. *Candida albicans* colonise, de façon mineure, la flore buccale de sujets sains sans provoquer d'infection. Mais lorsque ce champignon prolifère, il provoque une candidose qui se manifeste par une irritation des muqueuses buccales, associée à des rougeurs pouvant aller jusqu'à l'ulcération. Parfois, des tâches blanchâtres plus ou moins pâteuses peuvent siéger sur la langue, le palais et parfois l'oropharynx. Les candidoses buccales ou oropharyngées peuvent perturber l'élocution, les apports alimentaires et la qualité de vie.

Une prévalence de candidose oropharyngée est observée chez les enfants de moins de 18 mois due à une immaturité du système immunitaire et chez les individus ayant des flux réduits de salive entraînant une sécheresse buccale, comme les personnes âgées et les nourrissons entre la naissance et le 2^{ème} mois avant l'apparition de la salivation. Il existe également de nombreux facteurs favorisant la survenue des candidoses oropharyngées, tels que des facteurs locaux (comme le port prolongé d'appareils orthodontiques ou prosthétiques); des facteurs systémiques comme une immunovulnérabilité (par exemple associée au syndrome de Sjögren, une infection au VIH, un diabète non contrôlé, certains troubles endocriniens, une malnutrition ou malabsorption comme, par exemple une carence en vitamine B); et des effets secondaires liés à la médication (comme une antibiothérapie à large spectre d'action, l'utilisation systémique ou localisée de corticostéroïdes, l'administration de neuroleptiques, un traitement immunosuppresseur, une chimiothérapie ou une radiothérapie, un traitement par un inhibiteur de la pompe à protons (IPP) ou par un antihistaminique H2).

Les traitements disponibles pour les patients souffrant de candidose oropharyngée sont principalement limités à l'utilisation de molécules antifongiques. Le traitement local (au niveau de la bouche), sous forme de pastilles à sucer, de suspensions orales ou de gels buccaux, est préféré pour la plupart des patients. Cependant, dans certains cas, notamment chez les personnes immunodéprimées ou en cas d'échec du traitement local, de récidive, ou d'infection ancienne, un traitement systémique, administré par voie orale ou par injection, peut être nécessaire.

Les molécules antifongiques principalement utilisées, à ce jour, pour traiter les candidoses oropharyngées sont des macrolides polyènes (nystatine, amphotéricine B) et/ou des azoles (miconazole, fluconazole). Si ces médicaments sont généralement bien tolérés ils sont associés à des inconvénients importants. Ainsi, les polyènes, comme l'amphotéricine B, qui sont efficaces contre la plupart des isolats de *Candida,* ont tendance à avoir une toxicité rénale importante, et leur association avec d'autres médicaments comme les hypokaliémiants ou néphrotoxiques doit être évitée. Les antifongiques azolés, quant à eux, présentent des effets indésirables communs tels que l'hépatotoxicité (cytolyse, cholestase) et les troubles digestifs (nausées, vomissements, diarrhées). En cas de grossesse, les antifongiques de type triazole (comme le fluconazole) sont contre-indiqués, car ils sont tératogènes. De plus, les azoles étant tous, à des degrés variables, des inhibiteurs enzymatiques des isoenzymes du CYP450, ils sont à l'origine de nombreuses interactions médicamenteuses. Ils subissent par ailleurs eux-mêmes des phénomènes de métabolisation et sont donc également la cible d'interactions médicamenteuses.

Enfin, un problème d'envergure lié à l'utilisation de ces agents antifongiques est l'émergence grandissante de souches de *Candida albicans* résistantes (Kelly et al., Lancet, 1996, 348: 1523-1524). De plus, comme les antifongiques disponibles sont dirigés soit contre la partie "ergostérol" de la membrane fongique (dans le cas des polyènes) soit contre les enzymes impliqués dans la biosynthèse de cet ergostérol (dans le cas des azoles), il existe également un risque de résistance croisée (Kelly et al., FEBS Lett., 1997, 400: 80-82; White et al., Clin. Microbiol. Rev., 1998, 11: 382-402).

Il y a donc toujours un besoin de disposer de nouvelles stratégies pour le traitement et/ou la prévention des candidoses buccales ou oropharyngées

### Résumé de l'Invention

Les présents Inventeurs ont trouvé de manière surprenante que la souche de levure *Saccharomyces cerevisiae* déposée le 17 octobre 2007 auprès de la CNCM (Collection Nationale de Cultures de Microorganismes, 25 rue du Docteur Roux, 75724 Paris Cedex 15, France) sous le numéro I-3856 avait la capacité de réduire de manière efficace la charge de *Candida albicans* dans la cavité buccale d'un modèle animal murin de candidose oropharyngée et empêchait ainsi la propagation de l'infection à l'œsophage, à l'estomac et à l'intestin grêle. L'inhibition de la croissance fongique de *Candida albicans* dans la cavité buccale a été observée, que la levure *Saccharomyces cerevisiae* I-3856 utilisée dans le traitement des souris se trouve sous forme de levure vivante ou sous forme de levure inactivée. Une inhibition de la croissance de *Candida albicans* dans la cavité buccale a également été observée lorsque des parois cellulaires de la levure *Saccharomyces cerevisiae* I-3856 ont été utilisées dans le traitement des souris. L'effet bénéfique de *Saccharomyces cerevisiae* I-3856 sous forme vivante combine un effet mécanique *via* une forte agrégation de *Candida albicans* et un effet biologique *via* la modulation des facteurs de virulence de *Candida albicans,* tels que l'inhibition des adhésines ou l'inhibition de la transition hyphale qui empêchent l'adhésion des cellules fongiques aux cellules épithéliales buccales, tandis que l'effet de *Saccharomyces cerevisiae* I-3856 sous forme de levure inactivée et de parois cellulaires est principalement dû à un seul effet mécanique *via* une forte agrégation de *Candida albicans* qui empêche l'adhésion des cellules fongiques aux cellules épithéliales buccales. Tous ces effets induisent une accélération de l'élimination de *Candida albicans,* ce qui conduit à une faible réponse inflammatoire, voire même une absence de réponse inflammatoire.

La présente invention concerne donc la souche de levure *Saccharomyces cerevisiae* déposée, le 17 octobre 2007, auprès de la CNCM sous le numéro I-3856, pour utilisation dans la prévention et/ou le traitement d'une candidose oropharyngée.

Dans certains modes de réalisation, la souche de levure *Saccharomyces cerevisiae* numéro I-3856 utilisée dans la méthode de prévention et/ou de traitement d'une candidose oropharyngée est sous forme vivante ou inactive.

Dans certains modes de réalisation, la souche de levure *Saccharomyces cerevisiae* numéro I-3856 utilisée dans la méthode de prévention et/ou de traitement d'une candidose oropharyngée est sous forme de levure sèche, en particulier de levure sèche active.

Dans certains modes de réalisation, la souche de levure *Saccharomyces cerevisiae* numéro I-3856 utilisée dans la méthode de prévention et/ou de traitement d'une candidose oropharyngée est sous forme fractionnée. La forme fractionnée peut être choisie parmi les parois cellulaires de ladite levure, les β-glucanes pariétaux de ladite levure, les mannoprotéines pariétales de ladite levure, les extraits de ladite levure, et l'une quelconque de leurs combinaisons.

La présente invention concerne également une composition pharmaceutique pour son utilisation dans la prévention et/ou le traitement d'une candidose oropharyngée, la composition pharmaceutique comprenant une quantité efficace de la souche de levure *Saccharomyces cerevisiae* numéro I-3856, et au moins un excipient physiologiquement acceptable. La souche de levure *Saccharomyces cerevisiae* numéro I-3856, présente dans la composition pharmaceutique peut être sous n'importe quelle forme définie plus haut.

Dans certains modes de réalisation, la composition pharmaceutique est destinée à une administration topique ou à une administration par voie orale.

Dans certains modes de réalisation, la composition pharmaceutique comprend en outre au moins un principe actif pharmaceutique supplémentaire ayant une activité apaisante, anti-irritante, analgésique, antalgique, anti-inflammatoire, cicatrisante, antibiotique, antipyrétique, ou antifongique.

Dans certains modes de réalisation, la composition pharmaceutique se présente sous forme d'un dentifrice, d'un bain de bouche, d'un spray oral, d'une crème ou d'un gel oral, d'une feuille orodispersible, de pastilles, d'une poudre qui peut être saupoudrée directement dans la cavité buccale, d'une fiole avec bouchon à bouton poussoir, d'un stick orodispersible, ou d'un stick à diluer dans l'eau.

Dans certains modes de réalisation, la candidose oropharyngée qui est destinée à être prévenue ou traitée par la souche de levure *Saccharomyces cerevisiae* numéro I-3856, sous n'importe quelle forme définie plus haut, ou encore par la composition pharmaceutique définie plus haut, est un effet secondaire d'un traitement médical, en particulier une antibiothérapie à large spectre d'action, une utilisation systémique ou localisée de corticostéroïdes, une administration de neuroleptiques, un traitement immunosuppresseur, une chimiothérapie ou une radiothérapie, un traitement par un inhibiteur de la pompe à protons (IPP) ou par un antihistaminique H2.

Dans d'autres modes de réalisation, la candidose oropharyngée est présente ou susceptible de se développer chez un patient souffrant d'une immunovulnérabilité, en particulier une immunovulnérabilité associée à une maladie comme le syndrome de Sjögren, une infection au VIH, un diabète non contrôlé, certains troubles endocriniens, une malnutrition ou malabsorption comme, par exemple une carence en vitamine B.

Dans certains modes de réalisation, la candidose oropharyngée est présente chez un nourrisson ou chez une personne âgée.

La présente invention concerne aussi la souche de levure *Saccharomyces cerevisiae* numéro I-3856, sous n'importe quelle forme définie plus haut, ou la composition pharmaceutique définie plus haut pour utilisation dans une méthode pour prévenir ou inhiber la propagation de l'infection par *Candida* à l'œsophage, à l'estomac ou à l'intestin grêle chez un patient souffrant d'une candidose oropharyngée.

Une description plus détaillée de certains modes de réalisation préférés de l'invention est donnée ci-dessous.

### Description Détaillée de l'Invention

Comme mentionné plus haut, la présente invention concerne une souche de levure *Saccharomyces cerevisiae* ayant la capacité de réduire la charge de *Candida albicans* dans la cavité buccale et d'empêcher la propagation de l'infection à d'autres organes du système digestif, comme l'œsophage, l'estomac et l'intestin grêle. Cette souche est donc utile dans le traitement et/ou la prévention de candidoses oropharyngées.

### I - Souche de Levure Saccharomyces cerevisiae I-3856

L'expression "souche de levure" désigne une population relativement homogène de cellules de levure. Une souche de levure est obtenue à partir d'un clone, un clone étant une population de cellules obtenues à partir d'une seule cellule de levure.

La souche de levure *Saccharomyces cerevisiae* utilisée dans le contexte de la présente invention est la souche qui a été déposée le 17 octobre 2007, par le présent Déposant, à la CNCM (Collection Nationale de Cultures de Microorganismes, 25 rue du Docteur Roux, 75724 Paris Cedex 15, France) sous le numéro I-3856.

Cette souche de levure *Saccharomyces cerevisiae* a précédemment été décrite, par le présent Déposant lui-même, dans le document WO 2009/103884, où elle est décrite comme étant utile dans la prévention et/ou le traitement de pathologies, désordres ou troubles de l'intestin, y compris les inflammations provoquées par la colonisation intestinale par *Candida albicans;* ainsi que dans le document WO 2014/009656 et l'article de Cayzeele-Decherf et al. (Med. J. Obst. Gynec., 2017, 5(4): 1112), où elle est décrite comme étant efficace pour contrôler la prolifération vaginale de *Candida* et pour prévenir la récurrence des candidoses vaginales ou vulvovaginales. Il faut cependant rappeler que le comportement des champignons pathogènes et des probiotiques vis-à-vis de la muqueuse oropharyngée n'est pas transposable à celui adopté vis-à-vis de la muqueuse vaginale ou de la muqueuse intestinale, l'environnement de ces muqueuses étant notamment différent.

Dans le contexte de la présente invention, la souche *Saccharomyces cerevisiae* numéro I-3856 se trouve sous la forme de cellules de levure obtenues par culture (ou multiplication) de la souche de départ. La culture d'une souche *Saccharomyces cerevisiae* peut s'effectuer par n'importe quelle méthode appropriée. Les procédés de culture d'une souche *Saccharomyces cerevisiae* sont connus dans l'art, et l'homme du métier sait optimiser les conditions de culture pour chaque souche en fonction de sa nature. Les cellules de levure peuvent par exemple être obtenues par multiplication de la souche *Saccharomyces cerevisiae* numéro I-3856 dans un milieu de culture comme décrit dans l'ouvrage de référence "Yeast Technology", 2ème Edition, 1991, Reed and Nagodawithana, publié par Van Nostrand Reinhold (ISBN 0-442-31892-8).

Ainsi, par exemple, à l'échelle industrielle, des cellules de levure de *Saccharomyces cerevisiae* utilisables dans le contexte de la présente invention, peuvent être obtenues par un procédé comprenant les étapes suivantes:
- culture de la souche *Saccharomyces cerevisiae* numéro I-3856 dans un milieu de culture en plusieurs stades, d'abord en semi-anaérobiose, puis en aérobiose (milieu/atmosphère riche en oxygène) pour obtenir une multiplication des cellules de levure de départ; et
- séparation, par centrifugation, des cellules de levure ainsi produites pour obtenir une crème de levure liquide contenant entre 12% et 25% de matière sèche levure.

La levure ainsi obtenue est une levure vivante. Dans certains modes de réalisation, la souche *Saccharomyces cerevisiae* numéro I-3856 utilisée dans le contexte de l'invention est donc sous forme de levure vivante. Le terme "levure vivante", qui est synonyme de "levure active", désigne une population de cellules de levure qui sont métaboliquement actives.

La souche de levure I-3856 vivante utilisée dans le contexte de la présente invention se présente sous forme d'une levure sèche. Une levure sèche est caractérisée par une teneur faible en eau, et comprend généralement un taux de matière sèche levure supérieur à 90%, de préférence un taux de matière sèche comprend entre 93% et 96%. Un des avantages d'une levure sèche est sa longue durée de conservation.

Ainsi le procédé de production de cellules de la levure *Saccharomyces cerevisiae* numéro I-3856 peut en outre comprendre une étape ultérieure de séchage des cellules, pour obtenir une levure sous forme sèche. Le séchage peut par exemple être un séchage par lyophilisation, un séchage par lit fluidisé ou un séchage par atomisation.

Dans certains modes de réalisation, la souche de levure I-3856 vivante, sèche se présente sous forme d'une levure sèche active ou d'une levure sèche instantanée.

Une levure sèche active peut être obtenue par déshydratation de la levure pressée ou liquide par l'action conjointe de la chaleur (à basse température) et d'une activité mécanique, qui permettent de transformer un produit pâteux (levure pressée ou liquide) en un produit sec, sous forme de sphérules. Par exemple, la levure sèche active est obtenue par extrusion et séchage en lit fluidisé d'une levure pressée ou d'une levure liquide.

Une levure sèche instantanée peut être obtenue par déshydratation de la levure pressée ou liquide par l'action d'un gradient d'air chaud qui permet de transformer un produit pâteux (levure pressée ou liquide) en de fins vermicelles secs. La levure sèche instantanée doit ensuite, pour rester stable, être conditionnée en absence d'oxygène.

Dans d'autres modes de réalisation, la souche *Saccharomyces cerevisiae* numéro I-3856 utilisée dans le contexte de la présente invention est sous forme de levure inactive (par opposition à une levure vivante). Les termes "levure inactive" et "levure désactivée", qui sont ici utilisés indifféremment, désignent une levure qui n'est plus vivante (donc une levure morte), c'est-à-dire une levure dont le métabolisme est irrémédiablement arrêté.

Une levure inactive selon l'invention peut être obtenue par n'importe quelle méthode appropriée. Les techniques appropriées bien connues de l'homme du métier incluent un traitement thermique de la levure, un traitement consistant à soumettre la levure à plusieurs cycles de congélation et décongélation successives, un traitement par irradiation, un traitement par atomisation ou une combinaison quelconque de ces traitements. Une levure inactive se trouve généralement sous forme sèche.

Dans un autre mode de réalisation, c'est un dérivé ou une fraction de cellules de *Saccharomyces cerevisiae* numéro I-3856 qui est utilisé dans le contexte de la présente invention, ce dérivé ou cette fraction étant choisi dans le groupe constitué par les parois desdites cellules de levure, les glucanes pariétaux desdites cellules de levure, les mannoprotéines pariétales desdites cellules de levure, un filtrat ou extrait desdites cellules de levure et l'une quelconque de leurs combinaisons.

Les termes "parois cellulaires de levure" et "écorces de levure" sont utilisés ici de façon interchangeable et désignent la fraction insoluble des cellules de levure, c'est-à-dire la paroi et la membrane plasmique de la levure.

Les termes "filtrat de levure" et "extrait de levure" sont utilisés ici de façon interchangeable, et désignent la fraction soluble des cellules de levure, c'est-à-dire tout ce qui n'est pas paroi et membrane plasmique de la levure.

De façon classique, les écorces de levure ou les extraits de levure sont obtenus par un procédé comprenant une étape d'autolyse ou d'hydrolyse enzymatique, essentiellement par des protéases, suivie d'une étape de séparation de la fraction soluble de la fraction insoluble, la fraction insoluble isolée correspondant aux écorces de levure et la fraction soluble correspondant à l'extrait de levure. La fraction insoluble et/ou la fraction soluble peuvent ensuite être séchées. Le procédé d'obtention des écorces de levure est tel qu'il conserve les polysaccharides de structure de la paroi cellulaire, c'est-à-dire les β-glucanes et les mannanes, ces mannanes étant sous forme de mannoprotéines. Les procédés d'obtention d'écorces de levure et d'extraits de levure sont connus dans l'art (voir par exemple l'ouvrage de référence "Yeast Technology", 2ème édition, 1991, G. Reed et T.W. Nogodawithana, publié par Van Nostrand Reinhold, New York, ISBN 0-442-31892-8).

Les écorces de levure peuvent se présenter sous forme liquide, sous forme sèche ou sous forme visqueuse. On considère qu'elles sont sous forme sèche lorsque leur teneur en matière sèche est d'au moins 85%. Au contraire, si leur teneur en matière sèche est inférieure à 20% en masse, on considère qu'elles sont sous forme liquide. A partir de 20% et en-dessous de 85% en masse de matière sèche, on considère que les écorces de levure sont sous forme visqueuse. Les écorces de levure sont de préférence utilisées sous forme sèche.

Un extrait de levure peut se présenter sous forme sèche, de préférence sous forme de poudre hydrosoluble fine, sous forme liquide ou sous forme de pâte. On considère que l'extrait de levure est sous forme sèche lorsque sa teneur en matière sèche est d'au moins 85%. Si sa teneur en matière sèche est inférieure à 70% en masse, on considère qu'il est sous forme liquide. A partir de 70% et en dessous de 85% en masse de matière sèche, on considère que l'extrait de levure est sous forme de pâte. L'extrait de levure utilisé est de préférence sous forme sèche, plus préférentiellement sous forme de poudre hydrosoluble fine. Un extrait de levure comprend majoritairement des matières protéiques, de préférence au moins 55 % de matières protéiques.

Le terme "β-glucanes pariétaux" désigne les β-glucanes de la paroi des cellules de levure, qui sont essentiellement des polymères de glucose dont les unités glucose de la chaîne principale sont reliées par des liaisons β-1,3 et dont les ramifications sont reliées par des liaisons β-1,6. Les β-glucanes de levure sont insolubles et présentent une viscosité faible. L'homme du métier sait comment extraire les β-glucanes de la paroi des cellules de levure. Une méthode commune comprend des extractions successives à chaud avec une base et avec un acide (comme l'acide acétique), suivies par des lavages aqueux pour éliminer tout composé soluble des écorces de levure, et la récupération du matériel insoluble constitué des β-glucanes pariétaux.

Le terme "mannoprotéines pariétales" désigne des polysaccharides de levure et plus précisément des copolymères d'oses neutres ou acides (à 5 ou 6 atomes de carbone), liés entre eux par des liaisons glycosidiques et liés à des protéines. Dans les parois de levure, les glucanes constituent un réseau sur lequel sont liés de manière covalente d'autres glucanes, la chitine, et les mannoprotéines. Les mannoprotéines sont fixées aux glucanes par l'intermédiaire d'un glycosylphosphate contenant une chaîne de 5 résidus mannose. L'homme du métier sait comment extraire les mannoprotéines de la paroi des cellules de levure. Une méthode commune comprend une digestion enzymatique des parois de levure avec une préparation de β-glucanases (par exemple la préparation industrielle GLUCANEX^{™}), suivie par la séparation de l'hydrolysat par centrifugation, et une purification par ultrafiltration. Une autre méthode est basée sur une extraction chimique effectuée à chaud.

### II - Utilisations de la Souche de Levure Saccharomyces cerevisiae I-3856 dans le Traitement et/ou la Prévention de Candidoses Oropharyngées

L'invention concerne donc la souche *Saccharomyces cerevisiae* numéro I-3856 (sous l'une quelconque des formes décrites ci-dessus) pour le traitement et/ou la prévention de candidoses oropharyngées. L'invention concerne également une méthode de traitement et/ou de prévention d'une candidose oropharyngée chez un sujet, la méthode comprenant une étape d'administration d'une quantité efficace de la souche *Saccharomyces cerevisiae* numéro I-3856 au sujet. L'invention concerne aussi l'utilisation de la souche *Saccharomyces cerevisiae* numéro I-3856 pour la fabrication d'un médicament pour le traitement et/ou la prévention de candidoses oropharyngées.

Dans le contexte de la présente invention, on entend par "traitement" une méthode qui a pour but : (1) de retarder ou prévenir le début d'une maladie ou d'une condition clinique; (2) de ralentir ou d'arrêter la progression, l'aggravation ou la détérioration des symptômes de la maladie; (3) d'apporter des améliorations des symptômes de la maladie; et/ou (4) de guérir la maladie. Un traitement peut être administré avant le début de la maladie, pour une action prophylactique (on parle alors de "prévention"), ou il peut être administré après initiation de la maladie, pour une action thérapeutique.

Le terme "sujet" désigne ici un mammifère, et plus particulièrement un être humain, qui a la possibilité d'être victime d'une infection buccale par *Candida* mais qui n'est pas forcément atteint d'une candidose oropharyngée. Le terme "sujet" ne désigne pas un âge particulier et englobe donc les nouveaux nés, les enfants, les adolescents, les adultes, et les personnes âgées. Le terme "patient" est parfois utilisé ici, à la place de "sujet", lorsque le sujet souffre (c'est-à-dire a été diagnostiqué comme souffrant) d'une candidose oropharyngée.

Le terme "candidose oropharyngée" désigne les lésions de la cavité orale/buccale et/ou de l'oropharynx induites par les champignons du genre *Candida*, en particulier *Candida albicans,* capables de se développer chez un hôte devenu susceptible à l'infection.

Il existe plusieurs formes cliniques de la candidose oropharyngée. Certains patients ne seront atteints que d'une seule forme clinique alors que d'autres seront atteints de plus d'une forme de candidoses des muqueuses orales. Ces formes incluent:
- la candidose buccale pseudomembraneuse, plus connue sous le nom de muguet, qui est caractérisée par la présence d'une substance blanchâtre adhérente ressemblant à du fromage cottage sur la muqueuse orale, les plaques étant caractéristiquement distribuées sur la muqueuse buccale, au palais et sur la surface dorsale de la langue. Frotter les plaques avec un gratte-langue ou avec une gaze sèche permet de les retirer. La muqueuse sous-jacente apparaît alors normale ou érythémateuse.
- la candidose buccale érythémateuse, qui est caractérisée par des plaques ou macules érythémateuses atrophiées généralisées et bien définies. Cette candidose est plus fréquente que la candidose pseudomembraneuse;
- la candidose buccale hyperplasique ou candidose kératosique, qui est caractérisée par des plaques blanchâtres qui ne disparaissent pas lorsqu'on les gratte;
- la cheilite angulaire ou perlèche, qui est caractérisée par des fissures, une desquamation et un érythème aux commissures de la bouche; et
- la glossite losangique médiane ou glossite médiane rhomboïde, qui est caractérisée par des tâches rouges ou par des tâches rouges et blanches au mis-dos de la langue, avant les papilles calciformes.

L'aspect clinique, souvent très évocateur, est suffisant au diagnostic de la candidose oropharyngée. La confirmation biologique par l'examen mycologique est parfois demandée devant un aspect clinique atypique ou devant des lésions persistantes malgré un traitement adapté. Elle s'effectue à l'aide d'un prélèvement par un écouvillon au niveau de la lésion (lésion blanchâtre, plaque érythémateuse, sillons d'une perlèche). L'examen direct du prélèvement recherche des pathogènes bourgeonnants et la présence de pseudo-filaments (forme pathogène). *Candida albicans* pousse en 24-48 heures sur des milieux spécifiques, permettant l'identification de l'agent pathogène avec une quantification du nombre de colonies.

Une méthode de traitement et/ou de prévention d'une candidose oropharyngée selon l'invention comprend l'administration, à un sujet, d'une quantité efficace de la souche *Saccharomyces cerevisiae* numéro I-3856 (sous une des formes décrites plus haut). La quantité efficace, qui peut être administrée en une ou plusieurs doses, peut être déterminée par le médecin. La quantité exacte à administrer peut varier d'un patient à un autre, en fonction de l'âge, du poids, de la condition générale du patient, de la gravité et/ou de l'étendue de la candidose oropharyngée, etc. La quantité efficace à administrer peut également varier en fonction de l'effet thérapeutique désiré (c'est-à-dire prévention d'une candidose oropharyngée ou traitement une candidose oropharyngée). La quantité efficace à administrer peut aussi varier en fonction de la voie d'administration choisie, par exemple topique ou systémique.

Par exemple, une dose quotidienne de levure I-3856 sous forme de levure vivante sèche peut être comprise entre 1.10⁷ à 1.10¹¹ UFC, et de préférence entre 1.10⁹ à 5.10¹⁰ UFC. Le terme UFC désigne une Unité Formant Colonie.

Par exemple, une dose quotidienne de levure I-3856 sous forme inactive peut être comprise entre 1 mg et 10 g, de préférence entre 100 mg et 5 g.

Par exemple, une dose quotidienne de parois cellulaires de la levure I-3856 peut être comprise entre 0,5 mg et 5 g, de préférence entre 50 mg et 2,5 g.

Une méthode de traitement selon l'invention peut être utilisée pour traiter un premier épisode de candidose oropharyngée ou bien une récidive. Dans un cas comme dans l'autre, le patient peut précédemment avoir été traité par un antifongique ou antimycosique habituel. Alternativement, la levure I-3856 peut être le premier traitement prescrit au patient.

Une méthode de traitement selon l'invention peut être utilisée pour éviter, réduire, inhiber ou empêcher la propagation d'une infection buccale ou oropharyngée par *Candida* à une autre partie du système digestif comme l'œsophage, l'estomac et l'intestin grêle.

Une méthode de traitement selon l'invention peut également être utilisée pour prévenir une candidose oropharyngée chez un patient, que la candidose soit un premier épisode ou une récidive. Cela peut être le cas de patients recevant un traitement médical connu comme favorisant la colonisation des muqueuses orales par *Candida,* tel qu'une antibiothérapie à large spectre d'action, l'utilisation systémique ou localisée de corticostéroïdes, l'administration de neuroleptiques, un traitement immunosuppresseur, une chimiothérapie ou une radiothérapie, un traitement par un inhibiteur de la pompe à protons (IPP) ou par un antihistaminique H2. Cela peut également être le cas de patients souffrant d'une immunovulnérabilité par exemple associée à une maladie comme le syndrome de Sjögren, une infection au VIH, un diabète non contrôlé, certains troubles endocriniens, une malnutrition ou malabsorption comme, par exemple une carence en vitamine B. Cela peut aussi être le cas de certaines personnes âgées.

Dans certains modes de réalisation, un traitement selon l'invention est administré seul. En d'autres termes, la levure I-3856 est le seul agent à être administré, à l'exception d'éventuels bains de bouche antiseptiques.

Dans d'autres modes de réalisation, un traitement selon l'invention est administré en combinaison avec une autre thérapie, par exemple avec l'administration d'un antifongique habituel comme un antifongique local (par exemple la nystatine en comprimés à sucer ou en solution orale ou l'amphotéricine B ou le miconazole en gel buccal) ou un antifongique systémique (par exemple le fluconazole ou le kétoconazole ou l'intraconazole en comprimés).

### III - Compositions Pharmaceutiques

Comme indiqué plus haut, la souche *Saccharomyces cerevisiae* numéro I-3856 peut être administrée comme telle (sous une des différentes formes décrites ci-dessus) ou sous la forme d'une préparation ou composition pharmaceutique en combinaison avec au moins un excipient physiologiquement acceptable. Ainsi, plus spécifiquement, une composition pharmaceutique selon l'invention comprend une quantité efficace de la souche de levure *Saccharomyces cerevisiae* numéro I-3856 et au moins un excipient physiologiquement acceptable. Une composition pharmaceutique selon l'invention peut être classée en tant que préparation pharmaceutique disponible sur ordonnance ou en vente libre.

Dans le contexte de la présente invention, on entend par "excipient physiologiquement acceptable" tout milieu ou additif qui n'interfère pas avec l'efficacité de l'activité biologique du principe actif (ici la souche *Saccharomyces cerevisiae*), et qui n'est pas excessivement toxique pour le patient ou sujet, aux concentrations auxquelles il est administré. Un excipient physiologiquement acceptable peut être un excipient approprié à l'administration aux mammifères, en particulier aux humains.

Les compositions pharmaceutiques selon la présente invention peuvent être administrées en utilisant toute combinaison de dosage et de voie d'administration efficace pour obtenir l'effet thérapeutique/prophylactique désiré. Comme déjà indiqué plus haut, la quantité exacte à administrer peut varier d'un patient à un autre, en fonction de l'âge, du poids, de la condition générale du patient, de la gravité et de l'étendue de l'infection à *Candida*, etc... La voie d'administration (topique ou systémique) peut être choisie en fonction de la gravité et de l'étendue de l'infection à *Candida* et/ou en fonction de l'âge et/ou de la santé du patient.

A titre d'exemple, l'invention a pour objet une composition pharmaceutique telle que définie ci-dessus pour une utilisation quotidienne de levure I-3856 sous forme de levure vivante sèche dans une quantité de 1.10⁷ UFC à 1.10¹¹ UFC, de préférence dans une quantité de 1.10⁹ UFC à 5.10¹⁰ UFC, la dose efficace journalière pouvant être administrée en une, deux ou trois fois.

A titre d'exemple, l'invention a pour objet une composition pharmaceutique telle que définie ci-dessus pour une utilisation quotidienne de levure I-3856 sous forme inactive dans une quantité comprise entre 1 mg et 10 g, de préférence entre 100 mg et 5 g, la dose efficace journalière pouvant être administrée en une, deux ou trois fois.

A titre d'exemple, l'invention a pour objet une composition pharmaceutique telle que définie ci-dessus pour une utilisation quotidienne de parois cellulaires de la levure I-3856 dans une quantité comprise entre 0,5 mg et 5 g, de préférence entre 50 mg et 2,5 g, la dose journalière pouvant être administrée en un, deux ou trois fois.

La formulation d'une composition pharmaceutique selon la présente invention peut varier en fonction de la voie d'administration et du dosage pour lesquels la composition est destinée à être utilisée. Après formulation avec au moins un excipient physiologiquement acceptable, une composition pharmaceutique de l'invention peut être sous toute forme appropriée pour l'administration à un mammifère, en particulier l'homme, par exemple sous la forme de tablettes, comprimés, dragées, capsules, perles sirops, émulsions, onguents, pâtes, gels, poudres, sachets, solutions injectables, etc. L'homme de l'art sait sélectionner les véhicules et excipients les plus appropriés à la préparation d'un type donné de formulation. Une composition selon l'invention peut en outre comprendre des additifs comme des conservateurs, des édulcorants, des arômes, des agents viscosants, des colorants, des agents humectants, des agents de désintégration, des accélérateurs d'absorption, des agents lubrifiants, etc.

Dans certains modes de réalisation, une composition pharmaceutique selon l'invention ne contient qu'un seul agent actif: la levure I-3856 (ou les parois cellulaires). Une telle composition pharmaceutique ne contient pas, en particulier, un autre probiotique ou une combinaison de probiotiques.

Dans d'autres modes de réalisation, une composition pharmaceutique selon l'invention contient, en outre, au moins un principe actif pharmaceutique supplémentaire (c'est-à-dire, en plus de la levure I-3856 ou des parois cellulaires). On entend par "principe actif pharmaceutique", tout composé ou substance dont l'administration a un effet thérapeutique ou dont l'administration a un effet bénéfique à la santé ou à la condition générale d'un patient ou d'un sujet auquel il est administré.

Ainsi, un principe actif pharmaceutique peut être actif contre l'infection des muqueuses orales par *Candida* que l'on veut prévenir ou traiter par administration de la composition pharmaceutique ; ou peut être actif contre une condition ou un symptôme associé à la candidose oropharyngée (par exemple, des douleurs ou de la fièvre) ; ou encore peut accroître la disponibilité et/ou l'activité du ou des principes actifs de la composition pharmaceutique.

Des exemples de principes actifs pharmaceutiques qui peuvent être présents dans une composition de la présente invention incluent, sans limitation, les principes actifs ayant une activité apaisante, anti-irritante, analgésique, antalgique, anti-inflammatoire, cicatrisante, antibiotique, antipyrétique, ou encore antifongique (sans effet sur la levure I-3856), etc....

A moins qu'ils ne soient définis d'une autre manière, tous les termes techniques et scientifiques utilisés dans la Description ont la même signification que celle couramment comprise par un spécialiste ordinaire du domaine auquel appartient cette invention. De même, toutes les publications, demandes de brevet, tous les brevets et toutes autres références mentionnés ici sont incorporés par référence.

### Exemples

Les exemples suivants décrivent certains modes de réalisation de la présente invention. Cependant, il est entendu que les exemples et les figures ne sont présentés qu'à titre illustratif et ne limitent en aucun cas la portée de l'invention.

### Légende des Figures

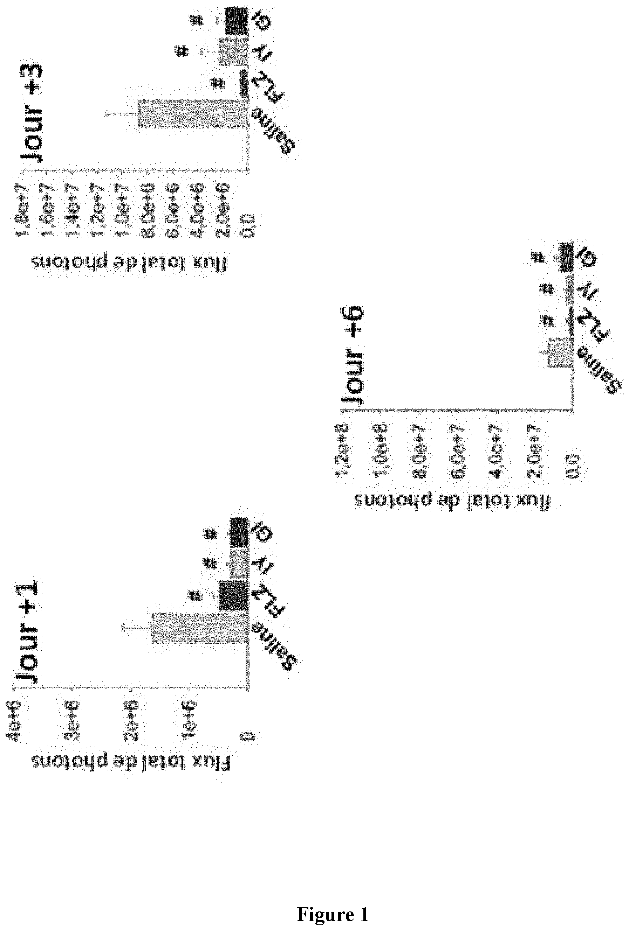

**Figure 1****:** Quantification de l'émission totale de flux de photons de la cavité buccale de souris infectées par BLI *Candida albicans* et traitées par différents composés, mesurée aux jours +1, +3 et +6 après infection. Les résultats présentent la moyenne ± SEM de 5 souris par groupe dans 3 expériences différentes. ≠, p<0.05 (souris infectées et traitées par FLU, GI ou IY vs. souris infectées et traitées par la solution saline).

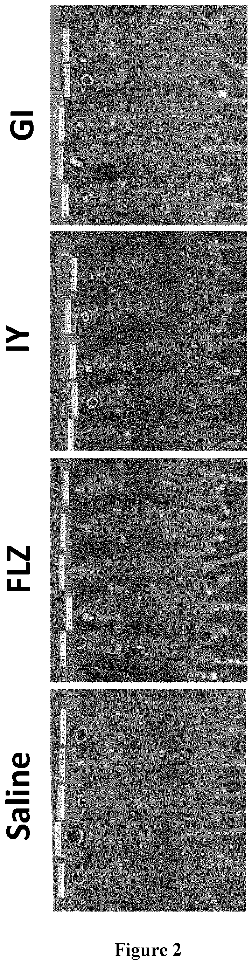

**Figure 2****:** Imagerie de souris *in vivo* infectées par BLI *Candida albicans* et traitées par différents composés, effectuée au jour +6 après infection.

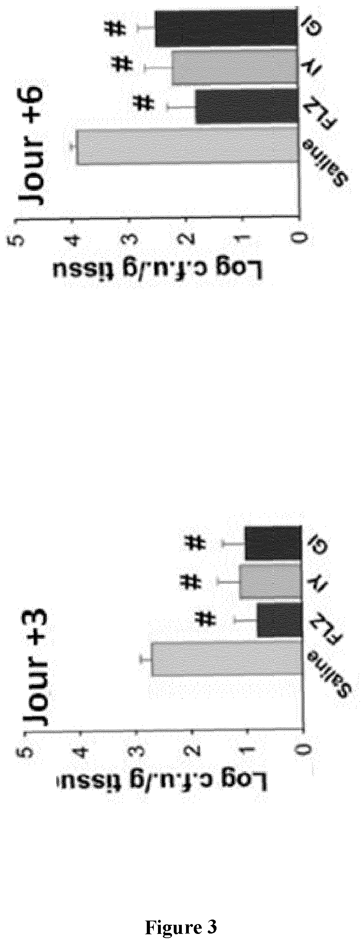

**Figure 3****:** Dénombrement CFU sur la langue de souris infectées par *Candida albicans* et traitées par différents composés, effectués aux jours +3 et +6 après infection. Les résultats présentent la moyenne ± SEM de 3 souris par groupe dans 3 expériences différentes. ≠, p<0.05 (souris infectées et traitées par FLU, GI ou IY vs. souris infectées et traitées par la solution saline).

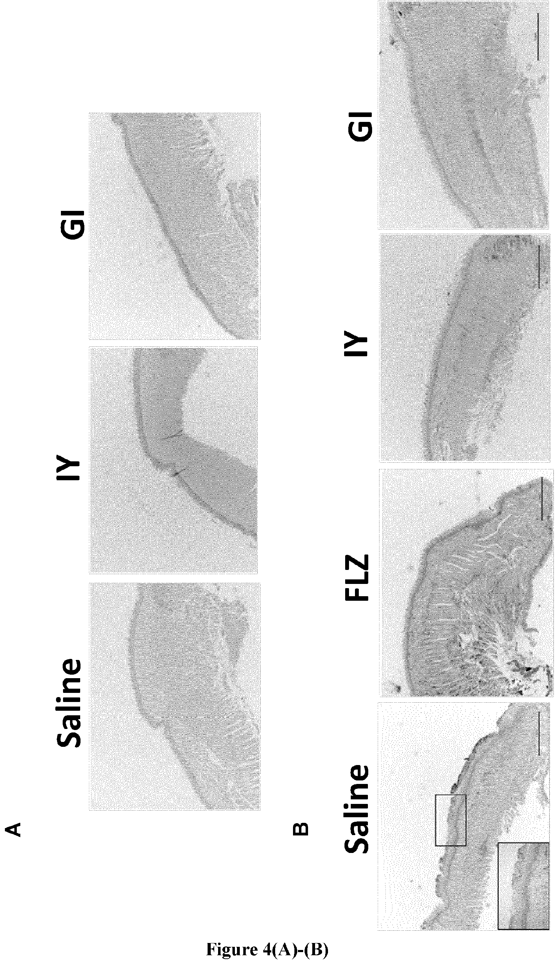

**Figure 4****:** Analyses histologiques au jour +8 **(A)** effectuées sur des souris non-infectées et traitées par différents composés et **(B)** effectuées sur des souris infectées par BLI *Candida albicans* et traitées par différents composés.

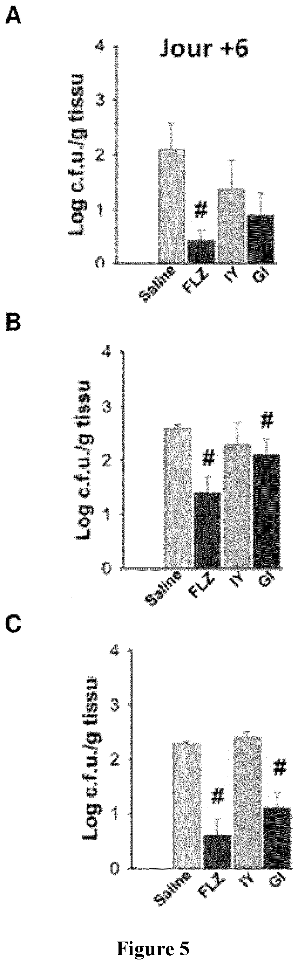

**Figure 5****:** Dénombrement CFU **(A)** dans l'œsophage, **(B)** dans l'estomac, et **(C)** dans le duodenum, de souris infectées par BLI *Candida albicans* et traitées par différents composés, effectués au jour +6 après infection. Les résultats présentent la moyenne ± SEM de 3 souris par groupe dans 3 expériences différentes. ≠, p<0.05 (souris infectées et traitées par FLU, GI ou IY vs. souris infectées et traitées par la solution saline).

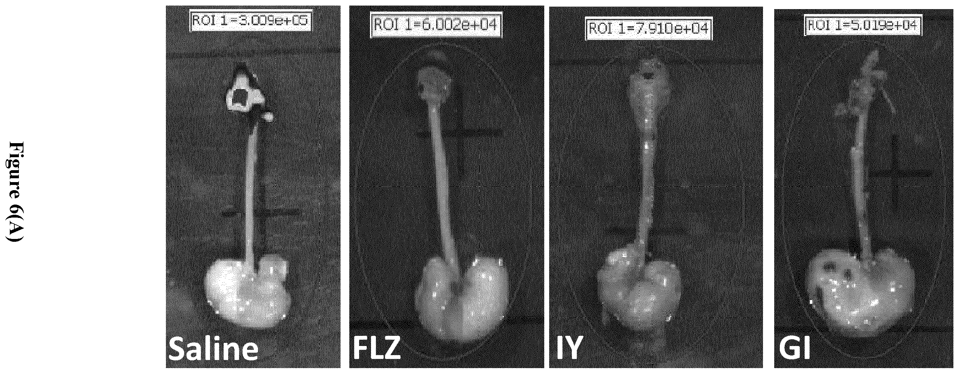

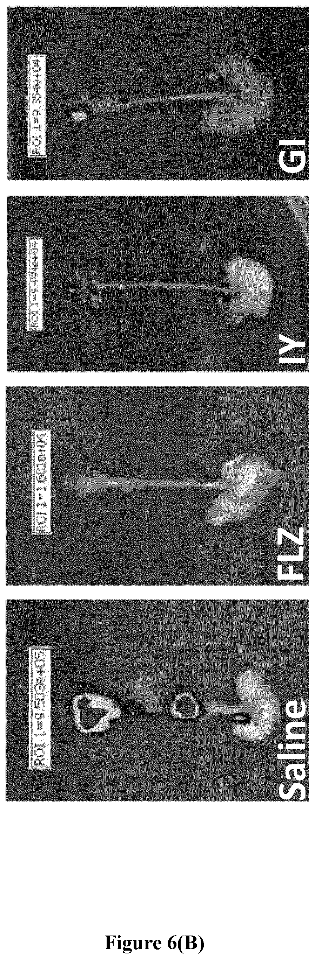

**Figure 6****:** Imagerie *ex vivo* de l'œsophage et de l'estomac de souris infectées par BLI *Candida albicans* et traitées par différents composés, effectué **(A)** au jour +6 et **(B)** au jour +8 après l'infection. Les résultats présentent la moyenne ± SEM de 5 souris par groupe dans 3 expériences différentes. ≠, p<0.05 (souris infectées et traitées par FLU, GI ou IY vs. souris infectées et traitées par la solution saline).

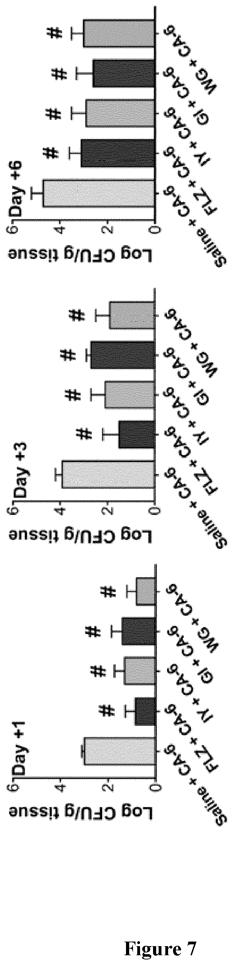

**Figure 7****: Dénombrements CFU sur la Langue.** La charge fongique de la langue des souris infectées et traitées par 10 µl de solution saline, ou de fluconazole (FLZ - 4 mg/ml), ou de IY, GI ou WG (tous à 100 mg/ml) aux jours +1, +2 et +3 post-infection, a été évaluée par un test CFU aux jours +1, +3 et +6 après infection de la langue. Les résultats présentent la moyenne ± SEM de 4 à 6 souris dans 2 expériences différentes. #, p<0,05 (souris infectées et traitées par FLZ, IY, GI ou WG *vs*. souris infectées et traitées avec une solution saline).

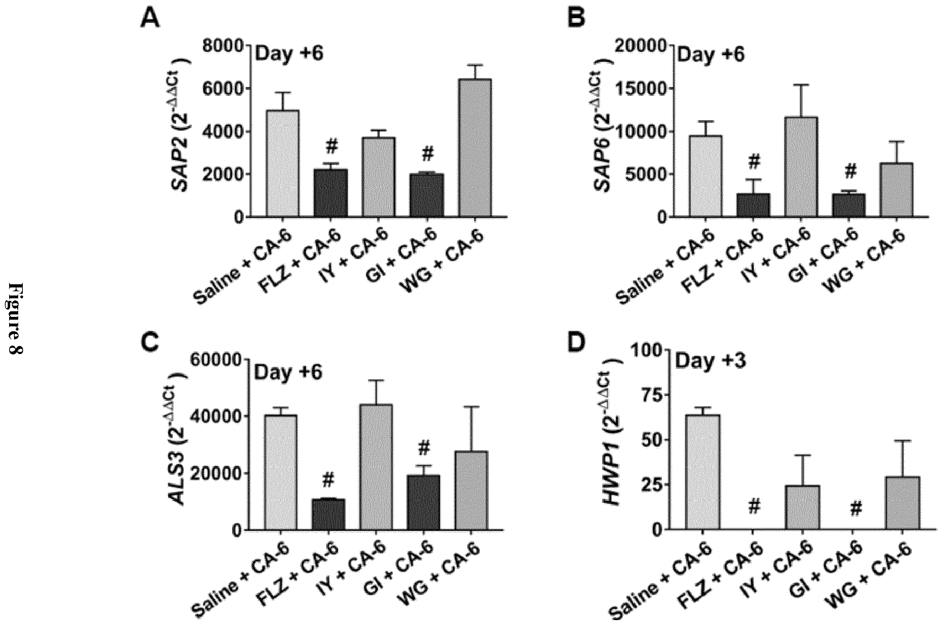

**Figure 8****: Effets de IY, GI et WG sur l'Expression de *SAP2, SAP6, ALS3* et *HWP1* au cours de la Candidose Oropharyngée.** L'expression des gènes **(A)** *SAP2,* **(B)** *SAP6,* **(C)** *ALS3* et **(D)** *HWP1* a été analysée dans des fractions cellulaires d'homogénats de langues de souris infectées et traitées par 10 µl de solution saline, ou de fluconazole (FLZ - 4 mg/ml), ou de IY, GI ou WG (tous à 100 mg/ml) aux jours +1, +2 et +3 post-infection. Aux jours 3+ et +6 post-infection, les homogénats de langue ont été centrifugés, puis les fractions cellulaires ont été lysées et l'ARN total a été extrait et rétro-transcrit en ADNc. Les gènes *SAP2, SAP6, ALS3* et *HWP1* de *Candida albicans* ont été détectés by PCR en temps réel et les quantités d'ADNc ont été reportées en 2^{-ΔΔCT} relativement aux transcrits de l'inoculum de *Candida albicans.* Les résultats présentent la moyenne ± SEM d'échantillons en triplicat de 4 à 6 souris dans 2 expériences différentes. #, p<0,05 (souris infectées et traitées par FLZ, IY, GI ou WG *vs*. souris infectées et traitées avec une solution saline).

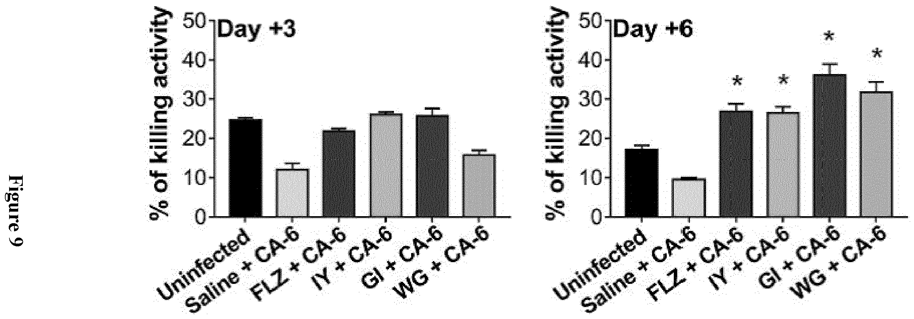

**Figure 9****: Activité Destructrice des Neutrophiles Péritonéaux.** L'activité destructrice des neutrophiles péritonéaux de souris non-infectées ou infectées et traitées par 10 µl de solution saline, ou de fluconazole (FLZ - 4 mg/ml), ou de IY, GI ou WG (tous à 100 mg/ml) aux jours +1, +2 et +3 post-infection, a été évaluée aux jours 3+ et +6 post-infection. Les neutrophiles péritonéaux (1×10⁶/ml) ont été incubés en présence de *Candida albicans* (CA-6) (1×10⁵/ml) pendant 2 heures. Les résultats présentent la moyenne ± SEM pour 4 à 6 souris de chaque groupe dans 2 expériences différentes. #, p<0,05 (souris infectées et traitées par FLZ, IY, GI ou WG *vs*. souris infectées et traitées avec une solution saline).

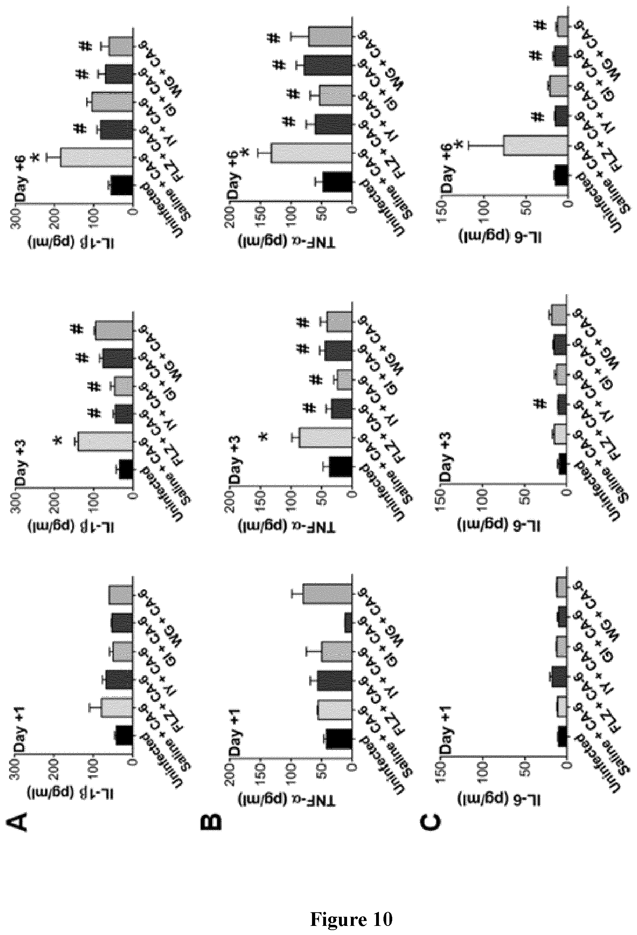

**Figure 10****: Production d'IL-1β, TNF-α et IL-6.** Les surnageants d'homogénats de langue de souris non-infectées ou infectées et traitées par 10 µl de solution saline, ou de fluconazole (FLZ - 4 mg/ml), ou de IY, GI ou WG (tous à 100 mg/ml) aux jours +1, +2 et +3 post-infection ont été testés, par ELISA, pour la présence **(A)** dIL-1β, **(B)** de TNF-α et **(C)** d'IL-6, aux jours +1, +3 et +6 post-infection. Les résultats présentent la moyenne ± SEM pour 4 à 6 souris de chaque groupe dans 2 expériences différentes. ^{∗}, p<0,05 (souris infectées et traitées avec une solution saline *vs*. souris non-infectées). #, p<0,05 (souris infectées et traitées par FLZ, IY, GI ou WG *vs*. souris infectées et traitées avec une solution saline).

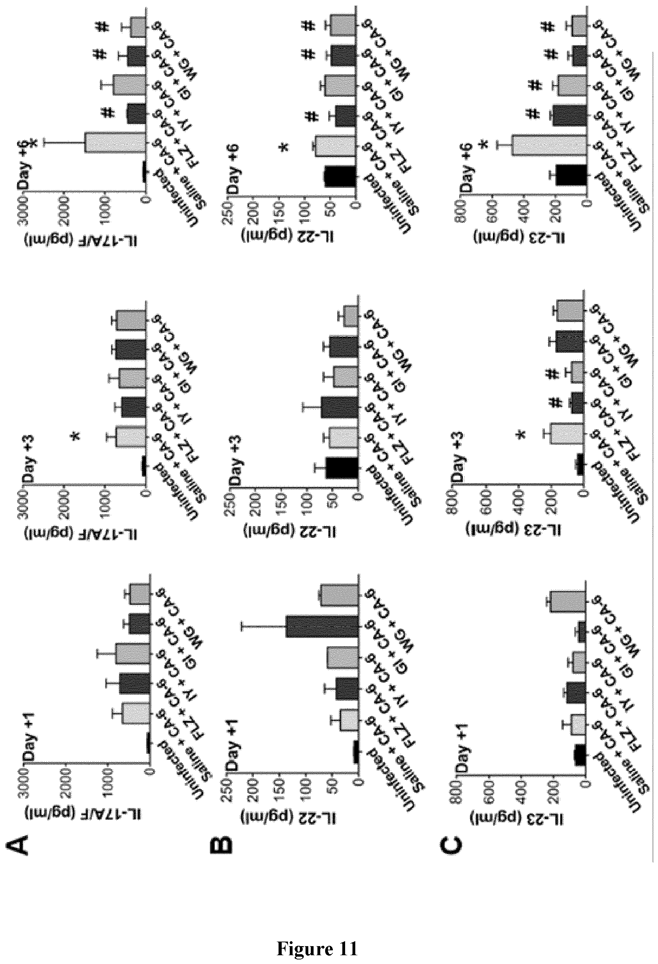

**Figure 11****: Production d'IL-17A/F, IL-22 et IL-23.** Les surnageants d'homogénats de langue de souris non-infectées ou infectées et traitées par 10 µl de solution saline, ou de fluconazole (FLZ - 4 mg/ml), ou de IY, GI ou WG (tous à 100 mg/ml) aux jours +1, +2 et +3 post-infection ont été testés, par ELISA, pour la présence **(A)** d'IL-17A/F, **(B)** d'IL-22 et **(C)** d'IL-23, aux jours +1, +3 et +6 post-infection. Les résultats présentent la moyenne ± SEM pour 4 à 6 souris de chaque groupe dans 2 expériences différentes. ^{∗}, p<0,05 (souris infectées et traitées avec une solution saline *vs*. souris non-infectées). #, p<0,05 (souris infectées et traitées par FLZ, IY, GI ou WG *vs*. souris infectées et traitées avec une solution saline).

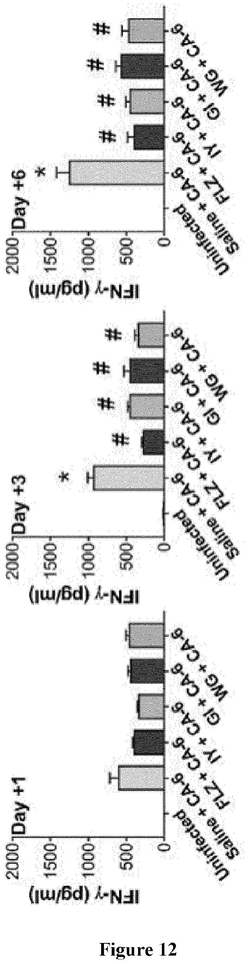

**Figure 12****: Production d'IFN-α**. Les surnageants d'homogénats de langue de souris non-infectées ou infectées et traitées par 10 µl de solution saline, ou de fluconazole (FLZ - 4 mg/ml), ou de IY, GI ou WG (tous à 100 mg/ml) aux jours +1, +2 et +3 post-infection ont été testés, par ELISA, pour la présence d'IFN-α aux jours +1, +3 et +6 post-infection. Les résultats présentent la moyenne ± SEM pour 4 à 6 souris de chaque groupe dans 2 expériences différentes. ^{∗}, p<0,05 (souris infectées et traitées avec une solution saline *vs*. souris non-infectées). #, p<0,05 (souris infectées et traitées par FLZ, IY, GI ou WG *vs*. souris infectées et traitées avec une solution saline).

### Exemple 1: Evaluation de l'Activité de Différents Produits Levure dans un Modèle Animal de Candidose Oropharyngée

### A. Matériels et Méthodes

***Modèle Animal de Candidose Oropharyngée.** Candida albicans* n'étant pas une espèce commensale chez la souris de laboratoire, la procédure développée par Solis et Filler (Nature Protoc., 2012, 7(4): 637-642) pour obtenir une infection reproductible qui mime la candidose oropharyngée pseusomenbraneuse chez l'homme a été utilisée sur des souris C57BL/6 wild-type. La procédure comprend l'injection d'acétate de cortisone qui rend la souris susceptible à l'infection orale par *Candida* et l'infection par *Candida albicans.*

Plus spécifiquement, des souris femelles C57BL/6 (Charles River, Calco, Italie), âgées de 6 à 8 semaines, ont été logées à l'animalerie de l'Université de Pérouse (Italie). Les souris ont été traitées avec 225 mg/kg d'acétate de cortisone (Sigma-Aldrich) tous les deux jours en commençant un jour avant l'infection et puis infectées avec une suspension de 1×10⁶/ml BLI *Candida albicans* comme précédemment décrit (Solis et Filler, Nature Protoc., 20125, 7: 637-642) sous anesthésie avec une injection sous-cutanée d'une mélange de Tiletamine/Zolazepam-Xylazine (50 mg/kg / 5 mg/kg) (Mosci et al., Virulence, 2013, 4: 250-254). La cavité buccale a été testée immédiatement avant l'infection pour confirmer l'absence préalable d'espèces de *Candida* (par un prélèvement buccal qui a été étalé sur de l'agar YPD avec du chloramphénicol (50 µg/ml) (tous deux de Sigma-Aldrich)).

Les souris ont été utilisées dans des conditions exemptes d'agents pathogènes spécifiques - conditions qui ont été contrôlées par des tests de sensibilité aux infections non désirées. Selon les normes de la Federation of European Laboratory Animal Science Associations, aucune infection n'a été détectée. Les procédures impliquant les animaux et leurs soins ont été menées conformément aux lois et normes nationales et internationales. Toutes les expériences sur les animaux ont été réalisées en accord avec la Directive Européenne 2010/63, la Convention Européenne sur la Protection des Animaux Vertébrés utilisés à des Fins Expérimentales ou à d'autres Fins Scientifiques et la loi nationale 116/92. Le protocole a été approuvé par le comité d'éthique de l'Université de Pérouse pour le soin et l'utilisation des animaux. Tous les animaux ont été logés dans l'animalerie de l'Université de Pérouse. Les souris ont été acclimatées pendant 1 semaine avant de commencer les expériences. Chaque cage contenait au maximum 5 souris qui ont reçu de la nourriture et de l'eau à volonté.

***Produits Testés.*** Ont été testées, dans cet exemple, la souche *Saccharomyces cerevisiae* I-3856 sous forme vivante (GI) et sous forme inactivée (IY). Après l'infection, les souris ont reçu une injection orale (10 µl) de solution saline (0,9% NaCl, contrôle négatif), de fluconazole (FLZ - l'agent antifongique de référence utilisé comme contrôle positif) (4 mg/ml) ou des produits levure GI et IY (tous l es deux à 100 mg/ml) aux jours +1, +2, +3 et +6.

***Candida.*** La souche de *Candida albicans* CA1398 portant le produit de fusion ACT1p-gLUC59 (gLUC59) a été utilisée. La culture de C. *albicans* a été maintenue par plusieurs passages sur un agar YPD (Y : extrait de levure, P : peptone et D : dextrose anhydre - tous de Sigma-Aldrich). Les cellules de champignon ont été récoltées en mettant en suspension une seule colonie de *Candida albicans* dans une solution saline, lavées deux fois, comptées à l'aide d'un hémocytomètre, et ajustées aux concentrations désirées.

### Evaluation de l'Infection par C. albicans dans la Cavité Orale.

***a.*** ***Test CFU.*** Le nombre de colonies de *Candida albicans* adhérant à la cavité orale a été évalué aux jours +3 et +6 après l'infection à *Candida albicans* en étalant les dilutions d'homogénats de langue sur des plaques CHROMAgar^{™} (milieu de croissance spécifique et sélectif pour *Candida*). Les colonies viables de *Candida albicans* ont ensuite été comptées après deux jours de culture à 30°C. Les résultats ont été exprimés en Log CFU/g tissu (ou Log UFC/g en français).
***b. Imagerie de BLI Candida dans la Cavité Buccale.*** Aux jours +1, +3 et +6 après l'infection, les souris ont reçu 10 µl (0.5 mg/ml dans un mélange 1/10 méthanol/H₂O) de coelenterazine (Synchem, OHM), et puis imagées en utilisant un système IVIS-200TM (Xenogen Inc.) sous anesthésie avec 2,5% d'isoflurane. Sur les images, l'émission totale de flux de photon de la cavité buccale (Region of Interest, ROI) de chaque souris a été quantifiée en utilisant le software Living ImageR. Aucune luminescence n'a été observée chez les souris non infectées recevant 10 µl de coelenterazine (données non montrées).
***c. Analyse Histologique.*** L'analyse histologique de la langue des souris a été effectuée au jour +8 après l'infection selon le protocole décrit par Mosci et al., Virulence, 2013, 4(3): 250-254.

### Progression de l'Infection à l'œsophage, l'Estomac et l'Intestin.

***a. Test CFU.*** Le nombre de colonies de *Candida albicans* qui se sont développées, après progression de l'infection, dans l'œsophage, l'estomac et le duodénum, a été évalué au jour +6 après l'infection à *Candida albicans* en étalant les dilutions d'homogénats des tissus/organes sur des plaques CHROMAgar^{™}. Les résultats ont été exprimés en Log CFU/g tissu (ou Log UFC/g en français).
***b. Imagerie de BLI Candida.*** Aux jours +6 et +8 après l'infection, les tractus gastriques ont été excisés et 10 µl (0.5 mg/ml dans un mélange 1/10 méthanol/H₂O) de coelenteratine (Synchem, OHM) ont été injectés à travers le pharynx dans la lumière de l'œsophage. L'œsophage et l'estomac des souris ont ensuite été imagés *ex vivo* en utilisant un système IVIS-200TM (Xenogen Inc.). Sur les images, l'émission totale de flux de photon (Region of Interest, ROI) pour chaque système œsophage/estomac de chaque souris a été quantifiée en utilisant le software Living ImageR.

***Analyses Statistiques.*** Les différences entre les souris infectées et traitées par FLU, GI ou IY et les souris infectées et traitées par la solution saline ont été évaluées avec le test U de Mann-Whitney. Une valeur de p<0.05 a été considéré comme significative.

### B. Résultats

***Evaluation de l'Infection par Candida albicans dans la Cavité Buccale.*** La Figure 1 et la Figure 2 montrent les résultats de l'imagerie *in vivo* des souris infectées et traitées par les différents composés. Ces résultats montrent que la souche *Saccharomyces cerevisiae* CNCM I-3856 aussi bien sous sa forme vivante (GI) que sous sa forme morte (IY) est capable de réduire fortement la charge fongique. L'effet bénéfique est similaire à celui obtenu en utilisant le fluconazole (FLU - contrôle positif).

Le nombre de colonies de *Candida albicans* adhérant à la langue des souris évalué aux jours +3 et +6 après l'infection à *Candida albicans* confirme que la souche *Saccharomyces cerevisiae* CNCM I-3856 aussi bien sous sa forme vivante (GI) que sous sa forme morte (IY) est capable de réduire significativement la charge fongique. L'effet bénéfique est similaire à celui obtenu en utilisant le fluconazole (FLU - contrôle positif). (Figure 3).

Les analyses histologiques effectuées sur des souris non-infectées et traitées par les deux produits levure (Figure 4(A)) montrent que la langue des souris non-infectées et traitées par GI et IY ne présente aucune lésion et qu'aucun recrutement de neutrophiles n'a été observé. Les analyses histologiques effectuées sur des souris infectées et traitées par les différents composés (Figure 4(B)) ont mis en évidence que la langue des souris infectées et traitées par la souche *Saccharomyces cerevisiae* CNCM I-3856 aussi bien sous sa forme vivante (GI) que sous sa forme morte (IY) ne présente aucune lésion induite par *Candida albicans.* L'effet bénéfique est similaire à celui obtenu en utilisant le fluconazole (FLU - contrôle positif).

***Progression de l'Infection à l'œsophage, l'Estomac et le Duodénum.*** La détermination du nombre de colonies de *Candida albicans* qui se sont développées, après progression de l'infection, dans l'œsophage (Figure 5(A)) dans l'estomac (Figure 5(B)) et dans le duodénum (Figure 5(C)) a été évaluée au jour +6 après l'infection à *Candida albicans.* Les résultats obtenus montrent que la souche *Saccharomyces cerevisiae* CNCM I-3856 sous sa forme vivante (GI) est capable d'inhiber significativement le portage fongique tandis que sa forme morte (IY) montre une tendance à réduire le portage fongique dans l'œsophage et l'estomac. L'effet bénéfique de GI est similaire à celui obtenu en utilisant le fluconazole (FLU - contrôle positif). L'imagerie *ex vivo* de l'œsophage et de l'estomac aux jours +6 (Figure (6A)) et +8 (Figure 6(B)) après infection confirme que la souche *Saccharomyces cerevisiae* CNCM I-3856 sous sa forme vivante (GI) et à un degré moindre sous sa forme morte (IY) prévient la progression de l'infection dans le tractus digestif.

### C. Conclusions

Considérés collectivement, les résultats obtenus dans l'Exemple 1 montrent que la souche *Saccharomyces cerevisiae* CNCM I-3856 aussi bien sous sa forme vivante (GI) que sous sa forme morte (IY) est capable de réduire fortement la charge fongique prévenant ainsi sa propagation à l'œsophage et à l'estomac. Les effets bénéfiques sont similaires à ceux obtenus en utilisant le fluconazole (contrôle positif).

### Exemple 2: Effets de la Souche Saccharomyces Cerevisiae I-3856 sur la Réponse Immunitaire à l'Infection Oropharyngée par Candida albicans

La salive est l'un des mécanismes innés de défense contre l'infection orale par *Candida.* La salive forme une pellicule sur les dents et l'épithélium oral. Les principaux composants de cette pellicule sont les mucines et l'immunoglobuline A (IgA) qui peuvent agréger *Candida albicans,* qui se trouve éliminé par l'action d'avaler. De plus, la salive contient des agents bioactifs, comme l'histatine 5, le lysosyme, la lactoferrine et la calprotectine ayant des propriétés fongicides. Bien que ces agents soient présents dans la salive à des concentrations faibles, leurs effets combinés sont soit additifs soit synergiques. En outre, la combinaison des agents salivaires de défense et des effets dynamiques du flux de salive limite la colonisation, la prolifération et l'invasion de l'épithélium oral par *Candida albicans,* ce qui conduit à une résistance orale innée à *Candida albicans* (Feller et al., J. Oral. Pathol. Med., 2014, 43: 563-569).

Les neutrophiles et les cellules T jouent un rôle important dans l'immunité mucosale anti-*Candida.* Les neutrophiles phagocytent et tuent les cellules de *Candida* par des mécanismes oxydatifs (Moyes et al., Clin. Dev. Immunol., 2011, 346307). Les cellules T qui sont principalement impliquées dans la réponse orale à *Candida* sont les lymphocytes T auxiliaires (en anglais T helper, Th) Th 1 et Th17. Les cellules Th1 produisent de l'IFN-γ qui est un activateur efficace des neutrophiles (Gattoni et al., Clin. Ter., 2006, 157: 457-468). En présence d'IL-6, IL-1β et TGF-β, les cellules T se différencient en Th17 et parviennent à maturation par stimulation avec IL-23. Les cellules Th17 produisent IL-17A, IL-17F et IL-22. IL-17A et IL-17F stimulent les cellules épithéliales pour produire des peptides antimicrobiens et favorisent le recrutement et l'activation des neutrophiles, permettant ainsi l'élimination fongique. IL-22 présente des effets similaires à ceux d'IL-17 vis-à-vis des cellules épithéliales et limite la croissance fongique (Hebecker et al., Expert. Rev. Anti Infect. Ther., 2014, 12: 867-879; Moyes et al., Clin. Dev. Immunol., 2011, 346307).

Dans l'Exemple 1, il a été démontré que la souche *Saccharomyces cerevisiae* numéro I-3856, qu'elle soit sous forme vivante (GI) ou sous forme inactivée (IY), est capable de réduire la charge fongique buccale empêchant la propagation de l'infection par *Candida* à l'œsophage et l'estomac. Dans le présent Exemple, la capacité de WG (des parois cellulaires de la souche *Saccharomyces cerevisiae* CNCM I-3856) à réduire la charge fongique buccale a tout d'abord été évaluée, puis il a été déterminé si l'administration orale de IY, de GI et de WG est capable d'influencer les facteurs de virulence fongique et la réponse inflammatoire au cours de la candidose orophargyngée.

### A. Matériels et Méthodes

***Souche de Candida albicans et Conditions de Culture.*** La souche de *Candida albicans* (CA-6) hautement virulente a été utilisée (Bistoni et al., Infect. Immun., 1986, 51: 6668-674). La culture de *Candida albicans* a été maintenue par passages successifs sur de l'agar YPD (Y : extrait de levure, P : peptone et D : dextrose anhydre - tous de Sigma-Aldrich). Les cellules de champignon ont été récoltées en mettant en suspension une seule colonie de *Candida albicans* dans une solution saline, lavées deux fois, comptées à l'aide d'un hémocytomètre et ajustées aux concentrations désirées.

***Modèle Animal de Candidose Oropharyngée.*** Des souris femelles C57BL/6 (Charles River, Calco, Italie), âgées de 6 à 8 semaines, ont été logées à l'animalerie de l'Université de Pérouse (Italie). Les souris ont été traitées avec 225 mg/kg d'acétate de cortisone (Sigma-Aldrich) tous les deux jours en commençant un jour avant l'infection et puis infectées avec une suspension de 1×10⁶/ml *Candida albicans* (CA-6) comme précédemment décrit (Solis et Filler, Nature Protoc., 20125, 7: 637-642) sous anesthésie avec une injection sous-cutanée d'un mélange de Tiletamine/Zolazepam-Xylazine (50 mg/kg / 5 mg/kg) (Mosci et al., Virulence, 2013, 4: 250-254). La cavité buccale a été testée immédiatement avant l'infection pour confirmer l'absence préalable d'espèces de *Candida* (par un prélèvement buccal qui a été étalé sur de l'agar YPD avec du chloramphénicol (50 µg/ml) (tous deux de Sigma-Aldrich). Voir Exemple 1 pour les déclarations d'éthique.

***Produits Testés.*** Ont été testées, dans cet exemple, la souche *Saccharomyces cerevisiae* I-3856 sous forme vivante (GI) et sous forme inactivée (IY) mais aussi sous la forme de parois cellulaires (WG). Après l'infection, les souris ont reçu une injection orale (10 µl) d'une solution saline (0.9% NaCl, contrôle négatif), de fluconazole (FLZ, 4 mg/ml, contrôle positif) et d'IY, de GI ou de WG (tous à 100 mg/ml) aux jours +1, +2 et +3 post-infection.

***Test CFU.*** Voir Exemple 1 pour les conditions opératoires. La charge fongique a été déterminée aux jours +1, +3 et +6 post-infection.

***Analyse Quantitative de l'Expression des gènes SAP2, SAP6, ALS3 et HWP1**.* Des homogénats de langues de souris infectées oralement par *Candida albicans* et traitées comme décrit plus haut avec une solution saline, ou du fluconazole, ou avec IY, GI ou WG, ont été obtenus aux jours +1, +3 et +6 post-infection. Les homogénats de langues de souris ont été centrifugés à 3000 tours/minute pendant 5 minutes, puis les fractions cellulaires ont été lysées ave du Trizol (Life Technology).

L'ARN total a été extrait et retro-transcrit en utilisant la réaction de transcriptase reverse du virus de la leucémie murine de Moloney (M-MLV RT) selon les instructions du fabricant. La concentration en DNA complémentaire (DNAc) a été déterminée avec un spectrophotomètre. Les gènes *SAP2, SAP6, ALS3* et *HWP1* de *Candida albicans* ont été détectés par des amorces connues (Naglik et al., J. Med. Microbiol., 2006, 55: 1323-1327; Naglik et al., Microbiology, 2008, 154: 3266-3280; Roudbarmohammadi et al., Adv. Biomed. Res., 2016, 5: 105). Les réactions de PCR en temps réel ont été effectuées dans des plaques PCR à 96-puits avec du SYBR green (BioRad). Pour les réactions de PCR en temps réel, 200 ng d'ADNc ont été utilisés. Tous les échantillons ont été mesurés en triplicat. Les taux d'expression relatifs des gènes de *Candida* à différents temps post-infection ont été reportés en 2^{-ΔΔCT} relativement aux transcrits de l'inoculum de *Candida albicans* (Pericolini et al., Virulence, 2017, 8: 74-90)). Les conditions d'amplification utilisées sont les mêmes pour *SAP2, SAP6, ALS3* et *HWP1* : 3 minutes à 95°C, 40 cycles de 10 secondes à 95°C et 30 secondes à la température d'hybridation spécifique de l'amorce. Les expériences ont été effectuées avec un Eppendorf Mastercycler.

***Tests du Pouvoir Candidacide.*** Aux jours +3 et +6 post-infection, les neutrophiles péritonéaux de souris non-infectées et de souris infectées et traitées comme décrit plus haut, ont été collectés 18 heures après une injection intrépéritonéale de 0,5 ml de solution de 10% thioglycolate sans endotoxine (Difco).

L'activité de destruction des neutrophiles a été déterminée par un test d'inhibition de CFU. Brièvement, des neutrophiles (10⁵ cellules) dans une suspension de 0,1 ml par puits, ont été incubés dans une plaque 96-puits de culture tissulaire à fond plat avec 10⁴ cellules de *Candida albicans* (CA-6) dans 0,1 ml de RPMI contenant 5% de FBS et incubés pendant 2 heures à 37°C, en présence de 5% CO₂. Après incubation, les plaques ont été agitées vigoureusement et les cellules ont été lysées par ajout de Triton X-100 (0,1% dans de l'eau distillée, concentration finale de 0,01% dans chaque puits). Des dilutions en série ont été préparées avec de l'eau distillée à partir de chaque puits. Les échantillons ont été étalés sur du dextrose agar Sabouraud avec du chloramphénical (50 mg/ml) en triplicat et les values de CFU ont été évaluées après 24 heures d'incubation à 37°C. Les cultures dites de contrôle consistaient en *Candida albicans* (CA-6) incubé avec du RPMI-1640 contenant 5% de FCS sans cellules effectrices.

***Production de Cytokines.*** Des homogénats de langues, provenant de souris non-infectées ou infectées oralement et traitées comme décrit ci-dessus, ont été obtenus aux jours +1, +3 et +6 post-infection. Les homogénats de langues ont été centrifugés à 3000 tours/minute pendant 5 minutes et les surnageants ont été collectés et les taux d'IL-1β, de TNF-α, d'IL-6, d'IL-17A/F, d'IL-22, d'IL-23 et d'IFN-y ont été mesurés par des tests ELISA (eBioscience).

***Analyses Statistiques.*** Les résultats sont les moyennes ± SEM d'échantillons en duplicat ou triplicat de 4 à 6 souris pour chaque groupe dans deux expériences différentes. Les différences entre les souris infectées et traités par la solution saline et les souris non-infectées ou les souris infectées et traitées par FLU, GI, IY ou WG et les souris infectées et traitées par la solution saline ont été évaluées avec le test U de Mann-Whitney. Une valeur de *p*<*0,05* a été considérée comme significative.

### B. Résultats et Discussion

La capacité de WG (parois cellulaires de la souche *Saccharomyces cerevisiae* CNCM I-3856) a été tout d'abord évaluée pour son influence sur la charge de *Candida albicans* dans la cavité buccale. A cette fin, les souris ont été traitées avec IY, GI ou WG (tous à 100 mg/ml) aux jours +1, +2 et +3 après infection avec *Candida albicans* (10⁶/ml). Les valeurs de CFU des langues des souris ainsi traitées ont été évaluées aux jours +1, +3 et +6 après infection. Les résultats obtenus, qui sont présentés Figure 7, montrent que tous les composés testés, y compris WG, sont capables d'inhiber de manière significative la charge de *Candida albicans* et l'effet bénéfique est similaire à celui obtenu en utilisant le fluconazole (FLZ).

Ensuite, les présents Inventeurs ont déterminé si l'inhibition de la charge fongique était liée à l'inhibition de certains facteurs de virulence de *Candida albicans.* Ils ont donc déterminé l'expression de protéases aspartiques (Sap) qui sont impliquées dans l'invasion des tissus par *Candida.* La détermination des gènes *SAP2* et *SAP6* a été effectuée aux jours +1, +3 et +6 post-infection. Les résultats, présentés sur la Figure 8(A) et (B), montrent qu'il y a une inhibition significative de l'expression de *SAP2* et *SAP6* après traitement avec GI mais pas avec IY ou avec WG - l'effet ayant été observé 6 jours après infection.

*Candida albicans* exprime l'invasine Als3 qui se lie aux cellules épithéliales, résultant en une endocytose du champignon par invasion et pénétration active des cellules épithéliales produisant un dommage cellulaire et une libération de cytokines pro-inflammatoires (Naglik et al., Microbes Infect., 2011, 13: 963-976). Le gène *HWP1* (hyphal wall protein 1) de *Candida albicans* code pour une protéine de la paroi cellulaire fongique qui est nécessaire à la croissance hyphale et à l'adhésion du champignon aux cellules épithéliales (Orsi et al., Microb. Pathog., 2014, 69-70: 20-27). L'expression des deux gènes *ASL3* et *HWP1* s'est trouvée être réduite de manière significative après traitement avec GI mais pas avec IY ou avec WG (voir Figure 8(C) et (D)). Ces résultats suggèrent que l'inhibition de CFU par GI pourrait être due à une inhibition de l'adhésion de *Candida* aux cellules épithéliales ainsi qu'à l'inhibition de la transition levure-hyphale avec un dommage cellulaire conséquent réduit. Etant donné que IY et WG n'influencent pas l'expression génique de *Candida,* il y a d'autres mécanismes d'action responsables de la réduction de CFU observée avec ces deux composés.

Les neutrophiles sont des cellules immunitaires effectrices majeures dans la destruction fongique (Gazendam et al., Immunol. Rev., 2016, 273: 299-311). En conséquence, l'activité destructrice des neutrophiles a été examinée. Les résultats obtenus sont présentés sur la Figure 9. Ils montrent que l'activité destructrice des neutrophiles, qui est affaiblie au cours de l'infection avec *Candida albicans* par rapport aux souris non-infectées, est restaurée par traitement avec les produits levure testés. En particulier, 6 jours après infection, une forte augmentation de l'activité destructrice des neutrophiles a été observée chez les souris infectées et traitées par GI, IY et WG par comparaison aux souris infectées et non-traitées.

Au cours d'une candidose buccale, les cellules épithéliales, *via* la libération de cytokines pro-inflammatoires, induisent un recrutement de neutrophiles qui limitent l'étendue du dommage subi par les cellules épithéliales en accélérant l'élimination de *Candida* (Trautwein-Weidner et al., Mucosal. Immunol., 2015, 8: 221-231). La production des cytokines pro-inflammatoires IL-1β, TNF-α et IL-6 a été testée dans le système expérimental utilisé ici. Les résultats obtenus sont présentés sur la Figure 10. Ces résultats montrent qu'un jour après infection, aucun changement dans la production des cytokines pro-inflammatoires, telles qu'IL-1β, TNF-α et IL-6, n'a été observé chez les souris infectées et traitées ou non avec les différents composés par comparaison avec les souris non-infectées. Par contre, 3 jours après infection, les souris infectées présentent une augmentation significative de production d'IL-1β et de TNF-α, et le traitement avec les produits levure ou avec FLZ résulte en une inhibition importante de ces cytokines. A ce moment, la production d'IL-6 reste inchangée comparée aux souris non-traitées à l'exception de FLZ. Quand l'analyse des cytokines pro-inflammatoires a été réalisée 6 jours post-infection, une forte augmentation a été observée dans les surnageants de langues de souris infectées comparés aux surnageants de langues de souris non-infectées, et le traitement par GI, WG et FLZ a conduit à une diminution significative de la production de toutes les cytokines alors que IY a seulement été capable d'inhiber de façon significative la production de TNF-α (Figure 10).

Il est connu que IL-17, IL-22 et IL-23 jouent un rôle dans l'élimination des champignons au cours de la candidose buccale (Hebecker et al., Expert. Rev. Anti Infect. Ther., 2014, 12: 867-879; Moyes et al., Clin. Dev. Immunol., 2011, 346307). La présence des cytokines IL-17, IL-22 et IL-23 des cellules T a donc également été déterminée dans le présent système. Les résultats obtenus (Figure 11) montrent que, 6 jours après infection, la présence de toutes ces cytokines est plus importante dans les surnageants de langues des souris infectées que dans les surnageants de langues des souris non-infectées. Le traitement avec GI et WG a résulté en une inhibition significative de toutes les cytokines testées, tandis que IY n'a induit une diminution significative que dans le cas de la production d'IL-23.

Une augmentation d'IFN-y, une cytokine typique des cellules T, a également été observée dans les surnageants de langues de souris infectées 3 jours et 6 jours post-infection, et le traitement avec les composés testés a conduit à une forte réduction de la production d'IFN-y (voir Figure 12).

Considérés collectivement, ces résultats suggèrent que l'inhibition des cytokines pro-inflammatoires observée après traitement avec les composés testés pourraient être attribuée à une diminution de la charge fongique. Il est concevable qu'en inhibant plusieurs facteurs de virulence, comme les protéases aspartiques et les adhésines de *Candida,* GI parvient à inhiber la charge de *Candida* et la réponse inflammatoire. Dans les expériences présentées ici, IY et WG n'influencent apparemment pas les facteurs de virulence de *Candida,* mais ils induisent une forte réduction de la charge fongique. Il a précédemment été montré que IY est capable de produire une forte agrégation avec *Candida* (Pericolini et al., Virulence, 2017, 8: 74-90). Il est concevable que WG soit efficace par un mécanisme similaire. De plus, l'augmentation de l'activité destructrice des neutrophiles observée après traitement avec tous les composés testés pourrait également expliquer la forte diminution de la charge fongique.

### C. Conclusions

Les résultats obtenus démontrent que tous les produits levure testés (GI, IY et WG) sont capables d'inhiber la croissance fongique de *Candida* dans la cavité buccale. L'effet bénéfique de GI est au moins en partie dû à l'inhibition de l'adhésion de *Candida* aux cellules épithéliales *via* l'inhibition des adhésines de *Candida* et l'inhibition de la transition hyphale. Les résultats suggèrent que l'effet de WG et de IY est en particulier dû à un effet mécanique *via* la production d'une forte agrégation de *Candida* qui empêche l'adhésion de *Candida* aux cellules épithéliales. Tous ces effets induisent une accélération considérable de l'élimination de *Candida,* ce qui conduit à une réponse inflammatoire faible ou même absente. Il est intéressant de noter que, dans toutes les déterminations effectuées, la charge fongique obtenue après traitement avec les produits levure testés (GI, IY, et WG) était comparable à celle obtenue avec le fluconazole, l'antifongique standard utilisé dans le traitement des candidoses oropharyngées.

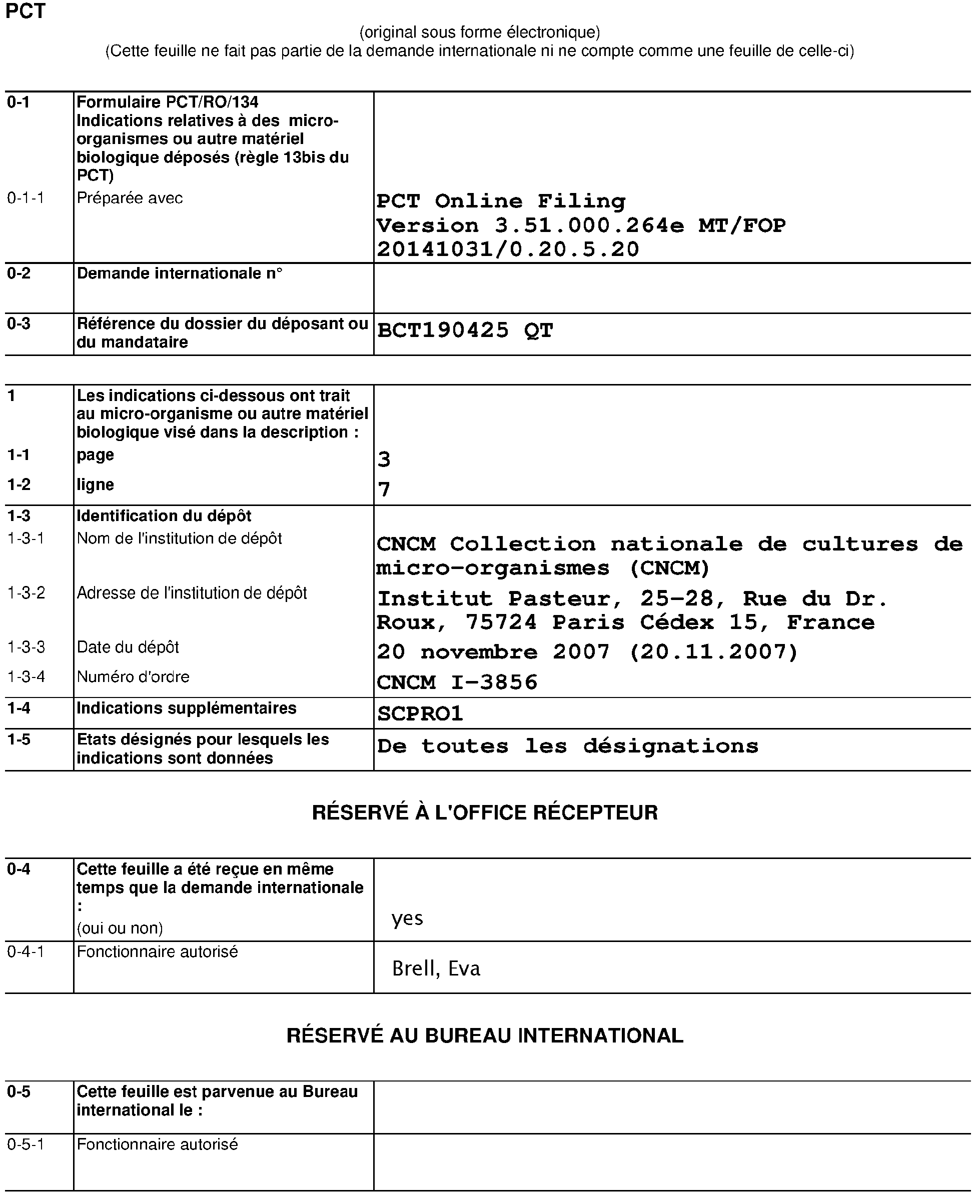

## Revendications

1. Souche de levure *Saccharomyces cerevisiae* déposée, le 17 octobre 2007, auprès de la CNCM sous le numéro I-3856, pour utilisation dans la prévention et/ou le traitement d'une candidose oropharyngée chez un sujet.

2. Souche de levure *Saccharomyces cerevisiae* numéro I-3856 pour utilisation selon la revendication 1, **caractérisée en ce que** la souche est sous forme vivante ou inactive.

3. Souche de levure *Saccharomyces cerevisiae* numéro I-3856 pour utilisation selon la revendication 1 ou la revendication 2, **caractérisée en ce que** la souche de levure se présente sous forme de levure sèche.

4. Souche de levure *Saccharomyces cerevisiae* numéro I-3856, pour utilisation selon la revendication 3, **caractérisée en ce que** la levure sous forme de levure sèche se présente sous forme de levure sèche active.

5. Souche de levure *Saccharomyces cerevisiae* numéro I-3856, pour utilisation selon la revendication 1, **caractérisée en ce que** la souche est sous forme fractionnée.

6. Souche de levure *Saccharomyces cerevisiae* numéro I-3856, pour utilisation selon la revendication 5, **caractérisée en ce que** la forme fractionnée est choisie parmi les parois cellulaires de ladite levure, les β-glucanes pariétaux de ladite levure, les mannoprotéines pariétales de ladite levure, les extraits de ladite levure, et l'une quelconque de leurs combinaisons.

7. Composition pharmaceutique pour son utilisation dans la prévention et/ou le traitement d'une candidose oropharyngée chez un sujet, la composition pharmaceutique comprenant une quantité efficace de la souche de levure *Saccharomyces cerevisiae* numéro I-3856 définie dans l'une quelconque des revendications 1 à 6, et au moins un excipient physiologiquement acceptable.

8. Composition pharmaceutique pour son utilisation selon la revendication 7, **caractérisée en ce que** la composition pharmaceutique est destinée à une administration topique ou à une administration par voie orale.

9. Composition pharmaceutique pour son utilisation selon la revendication 7 ou la revendication 8, **caractérisée en ce que** la composition pharmaceutique comprend en outre au moins un principe actif pharmaceutique supplémentaire ayant une activité apaisante, anti-irritante, analgésique, antalgique, anti-inflammatoire, cicatrisante, antibiotique, antipyrétique, ou antifongique.

10. Composition pharmaceutique pour son utilisation selon l'une quelconque des revendications 7 à 9, **caractérisée en ce que** la composition pharmaceutique se présente sous forme d'un dentifrice, d'un bain de bouche, d'un spray oral, d'une crème ou d'un gel oral, d'une feuille orodispersible, de pastilles, d'une poudre qui peut être saupoudrée directement dans la cavité buccale, d'un stick orodispersible, d'un stick à diluer dans l'eau, ou d'une fiole avec bouchon à bouton poussoir.

11. Souche de levure *Saccharomyces cerevisiae* numéro I-3856, ou parois cellulaires de ladite levure, pour utilisation selon l'une quelconque des revendications 1 à 6 ou composition pharmaceutique pour son utilisation selon l'une quelconque des revendications 7 à 10, **caractérisée**(s) en ce que la candidose oropharyngée est un effet secondaire d'un traitement médical.

12. Souche de levure *Saccharomyces cerevisiae* numéro I-3856, ou parois cellulaires de ladite levure, pour utilisation selon l'une quelconque des revendications 1 à 6 ou composition pharmaceutique pour son utilisation selon l'une quelconque des revendications 7 à 10, **caractérisée**(s) en ce que la candidose oropharyngée est présente ou susceptible de se développer chez un patient souffrant d'une immunovulnérabilité.

13. Souche de levure *Saccharomyces cerevisiae* numéro I-3856, ou parois cellulaires de ladite levure, pour utilisation selon l'une quelconque des revendications 1 à 6 ou composition pharmaceutique pour son utilisation selon l'une quelconque des revendications 7 à 10, **caractérisée**(s) en ce que la candidose oropharyngée est présente chez un nourrisson ou une personne âgée.

14. Souche de levure *Saccharomyces cerevisiae* numéro I-3856 définie dans l'une quelconque des revendications 1 à 6, ou parois cellulaires de ladite levure, ou composition pharmaceutique définie dans l'une quelconque des revendications 7 à 10 pour utilisation dans une méthode pour prévenir ou inhiber la propagation de l'infection par *Candida* à l'œsophage, à l'estomac ou à l'intestin grêle chez un patient souffrant d'une candidose oropharyngée.

## Patentansprüche

1. Hefestamm *Saccharomyces cerevisiae,* hinterlegt am 17. Oktober 2007 bei der CNCM unter der Nummer I-3856 zur Verwendung bei der Vorbeugung und/oder Behandlung von oropharyngealer Candidiasis bei einem Subjekt.

2. Hefestamm *Saccharomyces cerevisiae* Nummer I-3856 zur Verwendung nach Anspruch 1, **dadurch gekennzeichnet, dass** der Stamm in lebender oder inaktiver Form vorliegt.

3. Hefestamm *Saccharomyces cerevisiae* Nummer I-3856 zur Verwendung nach Anspruch 1 oder Anspruch 2, **dadurch gekennzeichnet, dass** der Hefestamm in Form von Trockenhefe vorliegt.

4. Hefestamm *Saccharomyces cerevisiae* Nummer I-3856 zur Verwendung nach Anspruch 3, **dadurch gekennzeichnet, dass** die Hefe in Form von Trockenhefe in Form von aktiver Trockenhefe vorliegt.

5. Hefestamm *Saccharomyces cerevisiae* Nummer I-3856 zur Verwendung nach Anspruch 1, **dadurch gekennzeichnet, dass** der Stamm in fraktionierter Form vorliegt.

6. Hefestamm *Saccharomyces cerevisiae* Nummer I-3856 zur Verwendung nach Anspruch 5, **dadurch gekennzeichnet, dass** die fraktionierte Form ausgewählt ist aus den Zellwänden der Hefe, den parietalen β-Glucanen der Hefe, den parietalen Mannoproteinen der Hefe, den Extrakten der Hefe und jede Kombination davon.

7. Pharmazeutische Zusammensetzung zur Verwendung bei der Vorbeugung und/oder Behandlung von oropharyngealer Candidiasis bei einem Subjekt, wobei die pharmazeutische Zusammensetzung eine wirksame Menge des Hefestamms *Saccharomyces cerevisiae* Nummer I-3856, definiert in einem der Ansprüche 1 bis 6, und mindestens einen physiologisch verträglichen Hilfsstoff umfasst.

8. Pharmazeutische Zusammensetzung zur Verwendung nach Anspruch 7, **dadurch gekennzeichnet, dass** die pharmazeutische Zusammensetzung zur topischen Verabreichung oder zur oralen Verabreichung bestimmt ist.

9. Pharmazeutische Zusammensetzung zur Verwendung nach Anspruch 7 oder Anspruch 8, **dadurch gekennzeichnet, dass** die pharmazeutische Zusammensetzung ferner mindestens einen zusätzlichen pharmazeutischen Wirkstoff mit beruhigender, reizlindernder, schmerzstillender, analgetischer, entzündungshemmender, heilender, antibiotischer, fiebersenkender, oder antimykotischer Wirkung umfasst.

10. Pharmazeutische Zusammensetzung zur Verwendung nach einem der Ansprüche 7 bis 9, **dadurch gekennzeichnet, dass** die pharmazeutische Zusammensetzung in Form einer Zahnpasta, eines Mundwassers, eines Mundsprays, einer oralen Creme oder eines oralen Gels, einer Schmelzfolie, von Lutschtabletten, eines Pulvers, das direkt in die Mundhöhle gestreut werden kann, eines Schmelzstäbchens, eines mit Wasser zu verdünnenden Stäbchens oder einer Ampulle mit Druckknopfverschluss vorliegt.

11. Hefestamm *Saccharomyces cerevisiae* Nummer I-3856 oder Zellwände dieser Hefe zur Verwendung nach einem der Ansprüche 1 bis 6 oder pharmazeutische Zusammensetzung zur Verwendung nach einem der Ansprüche 7 bis 10, **dadurch gekennzeichnet, dass** die oropharyngeale Candidiasis eine Nebenwirkung einer medizinischen Behandlung ist.

12. Hefestamm *Saccharomyces cerevisiae* Nummer I-3856 oder Zellwände dieser Hefe zur Verwendung nach einem der Ansprüche 1 bis 6 oder pharmazeutische Zusammensetzung zur Verwendung nach einem der Ansprüche 7 bis 10, **dadurch gekennzeichnet, dass** die oropharyngeale Candidiasis bei einem Patienten, der an Immunschwäche leidet, vorliegt oder dieser anfällig dafür ist.

13. Hefestamm *Saccharomyces cerevisiae* Nummer I-3856 oder Zellwände dieser Hefe zur Verwendung nach einem der Ansprüche 1 bis 6 oder pharmazeutische Zusammensetzung zur Verwendung nach einem der Ansprüche 7 bis 10, **dadurch gekennzeichnet, dass** oropharyngeale Candidiasis bei einem Säugling oder einer älteren Person vorliegt.

14. Hefestamm *Saccharomyces cerevisiae* Nummer I-3856, definiert in einem der Ansprüche 1 bis 6, oder Zellwände dieser Hefe, oder pharmazeutische Zusammensetzung, definiert in einem der Ansprüche 7 bis 10, zur Verwendung in einem Verfahren zur Vorbeugung oder Hemmung der Ausbreitung der *Candida* Infektion der Speiseröhre, des Magens oder des Dünndarms bei einem Patienten mit oropharyngealer Candidiasis.

## Claims

1. *Saccharomyces cerevisiae* yeast strain deposited with the CNCM on 17 October 2007 under number 1-3856, for use in the prevention and/or treatment of an oropharyngeal candidiasis in a subject.

2. *Saccharomyces cerevisiae* yeast strain number 1-3856 for use according to Claim 1, **characterized in that** the strain is in the live or the inactive form.

3. *Saccharomyces cerevisiae* yeast strain number 1-3856 for use according to Claim 1 or Claim 2, **characterized in that** the yeast strain is in the form of dry yeast.

4. *Saccharomyces cerevisiae* yeast strain number 1-3856, for use according to Claim 3, **characterized in that** the yeast in the form of dry yeast is in the form of active dry yeast.

5. *Saccharomyces cerevisiae* yeast strain number 1-3856, for use according to Claim 1, **characterized in that** the strain is in the fractionated form.

6. *Saccharomyces cerevisiae* yeast strain number 1-3856, for use according to Claim 5, **characterized in that** the fractionated form is selected from the cell walls of said yeast, the wall β-glucans of said yeast, the wall mannoproteins of said yeast, the extracts of said yeast, and any combination thereof.

7. Pharmaceutical composition for use in the prevention and/or treatment of an oropharyngeal candidiasis in a subject, the pharmaceutical composition comprising an effective amount of the *Saccharomyces cerevisiae* yeast strain number 1-3856 defined in any one of Claims 1 to 6, and at least one physiologically acceptable excipient.

8. Pharmaceutical composition for use according to Claim 7, **characterized in that** the pharmaceutical composition is intended for topical administration or for oral administration.

9. Pharmaceutical composition for use according to Claim 7 or Claim 8, **characterized in that** the pharmaceutical composition further comprises at least one additional pharmaceutical active ingredient having a soothing, anti-irritant, analgesic, painkilling, anti-inflammatory, healing, antibiotic, antipyretic, or antifungal activity.

10. Pharmaceutical composition for use according to any one of Claims 7 to 9, **characterized in that** the pharmaceutical composition is in the form of a toothpaste, a mouthwash, an oral spray, an oral cream or an oral gel, an orodispersible strip, lozenges, a powder which may be dusted directly into the oral cavity, an orodispersible stick, a stick to be diluted in water, or a vial with a push-button stopper.

11. *Saccharomyces cerevisiae* yeast strain number 1-3856, or cell walls of said yeast, for use according to any one of Claims 1 to 6 or pharmaceutical composition for use according to any one of Claims 7 to 10, **characterized in that** the oropharyngeal candidiasis is a side effect of a medical treatment.

12. *Saccharomyces cerevisiae* yeast strain number 1-3856, or cell walls of said yeast, for use according to any one of Claims 1 to 6 or pharmaceutical composition for use according to any one of Claims 7 to 10, **characterized in that** the oropharyngeal candidiasis is present or liable to develop in a patient suffering from an immunocompromised state.

13. *Saccharomyces cerevisiae* yeast strain number 1-3856, or cell walls of said yeast, for use according to any one of Claims 1 to 6 or pharmaceutical composition for use according to any one of Claims 7 to 10, **characterized in that** the oropharyngeal candidiasis is present in an infant or an elderly person.

14. *Saccharomyces cerevisiae* yeast strain number 1-3856 defined in any one of Claims 1 to 6, or cell walls of said yeast, or pharmaceutical composition defined in any one of Claims 7 to 10 for use in a method for preventing or inhibiting the spread of *Candida* infection to the oesophagus, the stomach or the small intestine in a patient with oropharyngeal candidiasis.
